# EUROPEAN PATENT SPECIFICATION

(11) **EP 4 739 692 B1**
(45) Date of publication and mention of the grant of the patent: **09.12.2026**
(21) Application number: 24739492.7
(22) Date of filing: 03.07.2024
(51) Int. Cl.: C07D 487/04, A61P 35/00, A61K 31/4985

(54) **SUBSTITUTED HEXAHYDRO-2H-PYRAZINO[1,2-A]PYRAZIN-1(6H)-ONE DERIVATIVES AS ACTIVATORS OF HUMAN CASEINOLYTIC PROTEASE P (HSCLPP)**
SUBSTITUIERTE HEXAHYDRO-2H-PYRAZINO[1,2-A PYRAZIN-1(6H)-ON DERIVATE ALS AKTIVATOREN DER MENSCHLICHEN CASEINOLYTISCHEN PROTEASE P (HSCLPP)
DÉRIVÉS D'HEXAHYDRO-2H-PYRAZINO[1,2-A!PYRAZIN-1(6H)-ONE SUBSTITUÉS COMME ACTIVATEURS DE LA PROTÉASE P CASÉINOLYTIQUE HUMAINE (HSCLPP)

(30) Priority: 03.07.2023 EP 23382682
(43) Date of publication of application: 13.05.2026
(73) Proprietor: Centro Atlantico del Medicamento S.A (Ceamed, S.A), 38204 Santa Cruz de Tenerife (ES); Fundación Canaria Instituto de Investigación Sanitaria de Canarias, 35019 Las Palmas de Gran Canaria (ES)
(72) Inventor: MCNAUGHTON-SMITH, Grant, 38740 Tenerife (ES); AYRA PLASENCIA, Jessel, 38009 Santa Cruz de Tenerife (ES); VELÁZQUEZ GARCÍA, Silvia Ascensión, 38007 Santa Cruz de Tenerife (ES); ESTÉVEZ SILVA, Héctor Martín, 38005 Santa Cruz de Tenerife (ES); ABREU GARCÍA, Andrea, 38108 San Cristóbal de La Laguna, Santa Cruz de Tenerife (ES); CHICO CAMPOS, María Atteneri, 38591 Güímar (ES); MACHÍN CONCEPCIÓN, Felix Manuel, 38320 Santa Cruz de Tenerife (ES)
(74) Representative: Manuel Illescas y Asociados, S.L.U.
(86) International application number: PCT/EP2024/068686
(87) International publication number: WO 2025/008381

(56) References cited:
- EP-A1- 4 174 068
- US-A1- 2022 089 596

## Description

### FIELD OF THE INVENTION

The present invention discloses compounds of formula (I), and pharmaceutical compositions containing formula (I), and their application as pharmaceuticals for the treatment of diseases. In particular, the invention discloses compounds of formula (I), their preparation and their use in treatments of a disease or condition responsive to activation of human caseinolytic protease P (HsClpP), or a bacterial caseinolytic protease P, such as SaClpP expressed in *Staphylococcus aureus.*

### BACKGROUND OF THE INVENTION

Proteolysis is an essential mechanism for eukaryotic cells. It allows them to maintain protein homeostasis, or modify protein levels, in order to adapt to their changing environment. This proteolysis is carried out by a group of specialized enzymes called proteases, among which is the caseinolytic protease P (ClpP). This protease ClpP, along with chaperone proteins, form a serine protease complex. Seven monomer ClpP units initially co-assemble to form a donut shaped entity. Two of these entities stack one upon the other to form a barrel shape with a central pore. The chaperones bind to one, or both, ends of this tetradecameric structure, to complete the ClpP-ClpPATPase protease system. The role of the chaperones is to bind damaged or misfolded proteins and feed them into the tetradecameric barrel shaped ClpP complex, which degrades them. In human cells, human ClpP (HsClpP) forms a complex with ClpXP chaperones, and the complex is located in the mitochondrial matrix. This complex participates in the maintenance of the mitochondrial proteome through the degradation of misfolded or dysfunctional proteins (1). This guarantees the proper functioning of the mitochondria, which regulates the bioenergetic activity of a cell (1).

Cancer cells need to generate higher quantities of proteins to support their prolonged growth/proliferation phases. To remove damaged or badly constructed proteins, that would be detrimental to these cells, they require elevated quantities of proteases to remove them. HsClpP expression levels have been reported to be considerably higher in several cancer cells compared to normal cells (2). Dysregulation of these well controlled proteolytic processes, by either inhibition or activation of their function, is detrimental to the well-being of these cells, and therefore maybe useful mechanism of action against cancer (3).

A few naturally occurring, and synthetic, compounds have been identified as activators of HsClpP (4-6). Activators of HsClpP induce significant conformational changes in the tetrameric structure, which lead to the loss of binding of the chaperones, and the stabilization of the axial pore in an open and active position. These combined events allow the HsClpP system to act independently of the chaperones, and to degrade a greater range of proteins. In human cells with elevated HsClpP levels, these agonists promote ClpP-mediated degradation of respiratory chain complexes, activating the JNK/c-Jun pathway and inducing an endoplasmic reticulum (ER) stress response. This response consequently leads to the activation of apoptopic pathways and causes growth arrest. Known activators of HsClpP, such as the acyldepsipeptides (ADEPs), TR-57, ZG-111, ONC-201 and ONC-206 have been reported to reduce cell viability in several human derived cancer cells including pancreatic, AML, glioma, glioblastoma, colorectal, ovarian and breast cancers Furthermore, several of these compounds have been reported to reduce tumour growth in *in vivo* models of cancers. Currently, the small molecules ONC-201 and ONC-206, are being studied in human clinical trials against several type of cancers.

Bacteria also possess significant levels of proteases including ClpP. Although bacteria do not possess mitochondria, bacteria versions of ClpP, such as *Staphylococcus aureus (SaClpP), Bacillus subtilis (BsClpP), Escherichia coli (EsClpP),* A. baumannii (AbClpP), K. pneumoniae (KpClpP) and P. aeruginosa (PaClpP) perform the same functions as HsClpP and maintain protein homeostasis. Genetic studies have established that the clpp gene, and genes encoding the AAA+ partners, are essential for virulence of several bacteria such as *S.aureus, Listeria monocytogenes* and *S.pnuemoniae.* Dysregulation of these well controlled proteolytic processes, by either inhibition or activation of their function, is detrimental to the well-being of these cells, and activators of bacterial versions of ClpP have been shown to have antibacterial properties. Recently the work reported by Wei, B., et al. (7) has demonstrated that small molecules that can selective activate SaClpP, over HsClpP, can possess promising antibacterial properties against wild-type, antibiotic resistant and clinical strains of S. aureus both in vitro and in vivo.

ClpP is also present in mycobacterium, such as M.tuberculosis (MtClpP). Genetic studies have also established that the Mtclpp gene is essential for the viability of *Mycobacterium tuberculosis.*

Herein we describe a family of substituted hexahydro-2H-pyrazino[1,2-a]pyrazin-1(6H)-one derivative compounds and their preparation. Depending on the chosen substituents these compounds activate HsClpP selectively or can activate both HsClpP and SaClpP. Compounds of the invention can reduce the cell viability and cell growth of several types of cancer cells and, those that are able to activate SaClpP, can also reduce the growth of S. aureus bacteria. US2022089596 relates to fused hydro-azines for the detection of ClpP as a biomarker of cancer or other diseases.

### BRIEF DESCRIPTION OF THE INVENTION

Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The present invention relates to compounds which act as activators of human caseinolytic protease P (HsClpP), wherein said compounds are characterized for having a formula (I), or a pharmaceutically acceptable salt thereof: 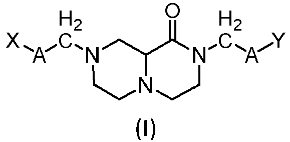 wherein;
A is independently selected from the group consisting of: C₁-C₆ alkyl or is absent;
X and Y are independently selected from the group consisting of C₁-C₆ haloalkyl, aromatic carbocyclic 6-membered ring, or an aromatic heterocyclic 5- or 6-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
R⁷ substituent is independently selected from the group consisting of: H, halogen, C₁-C₆ haloalkyl, -O-C₁-C₆ alkyl, -O-C₁-C₃ haloalkyl, cyano, C₁-C₆ alkyl and -C(O)OR⁸ ;
R⁸ is independently selected from the group consisting of: C₁-C₆ alkyl, C₃-C₆ cycloalkyl or C₁-C₆ aminoalkyl.

When A is absent X and/or Y are directly bound to the neighbouring CH₂ group.

The present invention also refers to a compound of formula (I), according to the present invention, or to a pharmaceutically acceptable salt thereof, for use as a medicament and, in particular, for use in the prevention and/or treatment of a disease or condition responsive activation of ClpP, in particular, for use in the prevention and/or treatment of cancer or bacterial infections.

Finally, the present invention also refers to a pharmaceutical composition comprising at least one compound of formula (I), according to the present invention, or a pharmaceutically acceptable salt thereof, and at least one pharmaceutically acceptable excipient, for use as a medicament and, in particular, for use in the prevention and/or treatment of a disease or condition responsive to activation of ClpP, particularly for use as medicaments in the prevention and/or treatment of cancer or bacterial infections.

### BRIEF DESCRIPTION OF THE FIGURES

**Figure 1****:** Representative comparison of the growth of yeast without HsClpP (a), and in yeast expressing HsClpP (b), in the presence of various concentrations of the known ClpP agonist ONC-206, and compounds of formula (I), (4), (6), (8), (9) and (12). Optical density was used as the measure of yeast growth. GI50 values represent the concentration of compound needed to reduce the growth by 50%. Selective activators of HsClpP will only reduce the growth of yeasts expressing HsClpP.
**Figure 2****:** Cell viability study in human derived MDA-MB-231 triple negative breast cancer cells. Compounds ONC-201, ONC-206, (1), (3), (5) and (12) were incubated with MDA-MB-231 cells are varying concentrations for 72 h. Cell viability was measure using the MTT assay. IC50 values represent the concentration of compound needed to reduce the viability by 50%.
**Figure 3****:** ONC-206, and compounds of the invention (12) and (13), reduce the growth of MDA-MB-231 cells, as measured by the SRB assay, in a concentration dependent manner.
**Figure 4****:** (a) Time-dependent growth, of established 4T1 tumours, treated with either vehicle, or compound (4), and (b) body weights during the experimental period.
**Figure 5****:** Normalized FITC-casein degradation data for compounds ONC-201, ONC-206 and compounds of the invention (1), (12) and (24) with the proteases HsClpP or SaClpP.
**Figure 6a****:** Thermal stability of the HsClpP protein (DMSO), and combinations of HsClpP with selected compounds of the invention. Graphs represent the d(Fluroresence)/dTime versus temperature.
**Figure 6b****:** ΔTm data for compounds (1), (12), (29), (30), (31), (32), ONC-201 and ONC-206 using HsClpP protein.
**Figure 7a****:** Thermal stability of the HsClpP protein (DMSO), and combinations of SaClpP with selected compounds of the invention. Graphs represent the d(Fluroresence)/dTime versus temperature.
**Figure 7b****:** ΔTm data for compounds (1), (12), (29), (30), (31), (32), ONC-201 and ONC-206 using SaClpP protein.
**Figure 8a****:** Cell viability study in human derived MDA-MB-231 triple negative breast cancer cells. Compounds (33), (34), (35) and (36) were with MDA-MB-231 cells are varying concentrations for 72 h. Cell viability was measure using the MTT assay. IC50 values represent the concentration of compound needed to reduce the viability by 50%.
**Figure 8b****:** Cell viability study in human derived DLD-1 colorectal cancer cells. Compounds (33), (34), (35) and (36) were with DLD-1 cells are varying concentrations for 72 h. Cell viability was measure using the MTT assay. IC50 values represent the concentration of compound needed to reduce the viability by 50%.
**Figure 9****:** Effect of different concentrations of compounds (29), (30), (31) and (32) and ONC-206 on the growth of S. aureus (ATCC25923) bacteria.

### DETAILED DESCRIPTION

The present invention refers to compounds of formula (I), or a pharmaceutically acceptable salt thereof, as disclosed herein, which activate the proteolytic functions of the human caseinolytic protease protein (HsClpP). Compounds of formula (I) according to the present invention are compounds comprising of a hexahydro-2H-pyrazino[1,2-a]pyrazin-1(6H)-one, core moiety, bearing two appendages at the non-bridgehead nitrogens, and are characterized by having a formula (I): 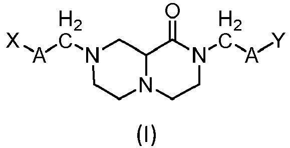 wherein;
A is independently selected from the group consisting of: C₁-C₆ alkyl or is absent;
X and Y are independently selected from the group consisting of C₁-C₆ haloalkyl, aromatic carbocyclic 6-membered ring, or an aromatic heterocyclic 5- or 6-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
R⁷ substituent is independently selected from the group consisting of: H, halogen, C₁-C₆ haloalkyl, -O-C₁-C₆ alkyl, -O-C₁-C₃ haloalkyl, cyano, C₁-C₆ alkyl and -C(O)OR⁸;
R⁸ is independently selected from the group consisting of: C₁-C₆ alkyl, C₃-C₆ cycloalkyl or C₁-C₆ aminoalkyl.

The present invention refers also to preferred compounds of formula (II), or a pharmaceutically acceptable salt thereof, as disclosed herein: 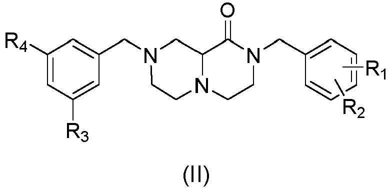 wherein;
R¹ and R² substituent are independently selected from the group consisting of: H, halogen and CF₃;
R³ and R⁴ substituent are independently selected from the group consisting of: halogen and CF₃.

Compounds of formula (II) not only activate the human form of ClpP but also the bacterial form ClpP expressed in S.aureus bacteria (SaClpP).

Present disclosure refers also to the pharmaceutically acceptable salts, solvates and polymorphs of the compounds of formula (I) described according to the present invention.

The term "pharmaceutical acceptable" is employed herein to refer to those compounds, materials, compositions, and/or dosage forms which, when contacted with the tissue of human beings or animals, possess a benefit/risk ratio greater than 1. As used herein "pharmaceutically acceptable salts" refers to derivatives of the disclosed compounds wherein the parent compound is modified by converting an existing acid or base moiety to its salt form. Examples of pharmaceutically acceptable salts include, but are not limited to:
- mineral or organic acid salts of basic residues such as amines. Examples of mineral acid salts of basic residues include hydrochloride, hydrobromide, hydroiodide, phosphate and sulfate salts. Examples of organic acid salts of basic residues include tartrate (e.g. (+)-L-tartrate), maleate, citrate, acetate, lactate, mesylate, methylsulfate and fumarate salts;
- alkali or organic salts of acidic residues such as carboxylic acids. Examples of alkali salts of acidic residues include sodium, potassium, calcium, magnesium, and ammonium salts. Examples of organic salts of acidic residues include L-argininate, N-methylglucaminate, cholinate, lysinate, benethaminate, diethylaminate, 2-diethylaminoethanol base, 4-(2-hydroxyethyl)morpholine base, 1-(2-hydroxyethyl)pyrrolidine base and trimethaminate base salts.

The salts may be formed by conventional means, such as by reacting the free base form of the compound with one or more equivalents of the appropriate acid in a solvent or medium in which the salt is insoluble, or in a solvent such as water which can be removed in vacuo or by freeze drying or by exchanging the anions of an existing salt for another anion using a suitable ion exchange resin.

The term "solvate" in accordance with this invention should be understood as meaning any form of the active compound in accordance with the invention in which said compound is bonded by a non-covalent bond to another molecule (normally a polar solvent), including especially hydrates and alcoholates.

The term "polymorph" refers to crystalline forms having the same chemical composition but different spatial arrangements of the molecules, atoms, and/or ions forming the crystal. Certain compounds of the present invention can exist in unsolvated forms as well as solvated forms, including hydrate forms. Certain compounds of the present invention may exist in multiple crystalline (polymorph) or amorphous forms.

The abbreviations used herein have their conventional meaning within the chemical and biological arts. For example, DMSO, dimethylsulfoxide; MeOH, methanol; RPMI medium, Roswell Park Memorial Institute medium; THF, tetrahydrofuran; DCM, dichloromethane; EtOAc, ethyl acetate, EtOH, ethanol; MTT, 3-(4,5-methyltiazol-2yl)-2,5diphenyl-tetrazolium bromide]; FBS, Fetal bovine serum; MgSO₄, Magnesium sulphate anhydrous; HCl, hydrochloric acid, H₂SO₄, sulfuric acid, AcOH, acetic acid; ATCC, American Type Culture Collection; DMEM, Dulbecco's Modified Eagle's Medium.

For the purposes of present invention, the term "comprises" includes any of the terms "consist of", or "consists essentially of". Accordingly, "comprises" may refer to a group of features A, B and C, which additionally may include other features, such as E and D, with the condition that said features do not render the claim unworkable, but said term "comprises" also includes the situation in which the group of features "consists of" or "consists essentially" of A, B and C.

The term "C₁-C₆ alkyl" refers to straight chain or branched chain hydrocarbon groups having from 1 to 6 carbon atoms. Examples include methyl, ethyl, propyl, isopropyl, butyl, isobutyl, sec-butyl, tert-butyl, pentyl, neopentyl and hexyl, preferably ethyl, propyl, isopropyl, n-butyl, isobutyl, sec-butyl, tert-butyl, pentyl, neopentyl and hexyl, more preferably ethyl, propyl, isopropyl, n-butyl, tert-butyl or pentyl.

The term "cycloalkyl group" refers to a non-aromatic carbocycle or cyclic hydrocarbon group. Examples include cyclopropyl, cyclobutyl, cyclopentyl, cyclopentenyl, cyclohexyl, cyclohexenyl, cycloheptyl, norbornyl and adamantly.

The term "C₁-C₆ aminoalkyl" refers to straight chain or branched chain hydrocarbon groups having from 1 to 6 carbon atoms and bearing an amino (-NH₂) or substituted amino group. Examples include CH₂NH₂, CH₂NHCH₃, CH₂N(CH₃)₃, CH₂NHCH₂CH₃, CH₂CH₂NH₂, CH₂CH₂NHCH₃, CH₂CH₂N(CH₃)₂, CH(NH₂)CH₃, CH(NHCH₃)CH₃, CH₂C[N(CH₃)₂]CH₃, CH₂CH(NH₂)CH₃, CH₂CH(NHCH₃)CH₃, CH₂CH₂CH₂NH₂.

The term "C₃-C₆ aminocycloalkyl" refers to cyclic hydrocarbon chain that bears an amino (-NH₂) or substituted amino group. Examples include cyclopropanamine, cyclobutanamine, cyclopentanamine, cyclohexanamine, N-methylcyclopropanamine, N-methylcyclobutanamine, N-methylcyclopentanamine, N-methylcyclohexanamine, N,N-dimethylcyclopropanamine, N,N-dimethylcyclobutanamine, N,N-dimethylcyclopentanamine, N,N-dimethylcyclohexanamine.

The terms "heterocyclic group" or "heterocycloalkyl group" refer both indistinctively to non-aromatic carbocycles having one or more ring forming heteroatoms such as N, O and S atoms. Heterocyclic groups may include monocyclic and polycyclic (e.g., having 2, 3 or 4 fused rings) systems as well as spirocycles. Example groups include morpholino, thiomorpholino, piperazinyl, tetrahydrofuranyl, tetrahydrothienyl, 2,3-dihydrobenzofuryl, piperidinyl and pyrrolidinyl. Ring forming carbon atoms and heteroatoms of a heterocyclic group can be optionally substituted by oxo or sulfido. Also included in the definition of heterocyclic group are moieties that have one or more aromatic rings fused (i.e., having a bond in common with) to the non-aromatic heterocyclic ring. Examples of heterocyclic groups include without limitation, phthalimidyl, naphthalimidyl, and benzo derivatives of heterocycles. The heterocyclic group can be attached through a ring-forming carbon atom or a ring-forming heteroatom. The number of carbon atoms present in the heterocyclic group can range from 1 to 20 and may include double or triple bonds.

The terms "aryl group" or "aromatic carbocyclic group" refer both indistinctively to single, polynuclear, conjugated or fused residues of cyclic aromatic hydrocarbons, including both substituted or unsubstituted versions thereof. Examples of "aryl groups" or "aromatic carbocycle groups" include: phenyl, biphenyl, naphthyl, tetrahydronaphthyl, anthracenyl, benzanthracenyl, phenylanthrenyl, nitrophenyl, chlorophenyl, bromophenyl, fluorophenyl, iodophenyl, benzo[d][1,3]dioxol-5-yl, alkylphenyls, alkoxyphenyl, hydroxyphenyl, aminophenyl, cyanophenyl and mercaptophenyl, preferably 3-nitrophenyl, 4-nitrophenyl, 3-fluorophenyl, 4-fluorophenyl, 3-iodophenyl, 4-iodophenyl, ethylphenyl, isopropylphenyl, 3-benzamide, 4-benzamide, aminophenyl, 3-cyanophenyl, 4-cyanophenyl and mercaptophenyl, more preferably 4-nitrophenyl, 4-cyanophenyl, 4-benzamide and 3-cyanophenyl.

The term "heteroaryl group" or "aromatic heterocyclic group" refers to an aromatic carbocycle having at least one heteroatom. As used herein, the term "heteroatom" is meant to include oxygen (O), nitrogen (N), sulphur (S), silicon (Si) and phosphorus (P); preferably, said heteroatom is S, N or O. Heteroaryl groups include monocyclic and polycyclic systems. Examples of heteroaryl groups include without limitation, pyridyl, pyrimidinyl, pyrazinyl, pyridazinyl, triazinyl, furyl, quinolyl, isoquinolyl, thienyl, imidazolyl, thiazolyl, indolyl, pyrolyl, benzofuryl, benzothienyl, isoxazolyl, pyrazolyl, triazolyl, tetrazolyl, indazolyl, carbazolyl, benzimidazolyl and indolinyl, preferably pyridyl, pyrimidinyl, pyrazinyl, pyridazinyl, triazinyl, furyl, quinolyl, isoquinolyl, imidazolyl, thiazolyl, indolyl, pyrolyl, benzofuryl, benzothienyl, isoxazolyl, pyrazolyl, triazolyl, tetrazolyl, indazolyl, carbazolyl, benzimidazolyl and indolinyl. In some embodiments, any ring forming N can be substituted by an oxo, to form an N-oxide.

The term "halogen" refers to fluorine, chlorine, bromine and iodine. The terms "amino", "azido", "nitro", "cyano" and "hydroxyl" respectively refer to -NH₂, -N₃, - NO₂, -CN and -OH groups attached to the remainder of the molecule.

For the purpose of present disclosure the dash "-" featured in a group listed is linked to the atom by which said group is attached to the reminder of the molecule. For example, for the purposes of the present disclosure, the group -OC(O)CH₃ is an ester group which is attached to the reminder of the molecule by the oxygen atom, whereas the group -C(O)OCH₃ is an ester group which is attached to the reminder of the molecule by the carbon atom of the carbonyl moiety. When the "-" is not indicated in a group, it may be linked to the reminder of the molecule by any of the atoms which are capable of forming a bond.

The term "substituted or unsubstituted" refers to a group that may or may not be further substituted with one or more groups selected from (C₁-C₆)alkyl, Si[(C₁-C₆)alkyl]₃, (C₃-C₈)cycloalkyl, (C₂-C₆)alkenyl, (C₂-C₆)alkynyl, aryl, heteraryl, heterocyclyl, halogen, (C₁-C₆)alkoxyl, amino, amido, azido, nitro, cyano, carboxy, sulphonamide, urea, sulphone, acyl, mercapto, alkylamino, alkylthio and hydroxyl. Preferably, said term "substituted or unsubstituted" refers to a group that may or may not be further substituted with one or more groups selected from (C₁-C₆)alkyl, Si(C₁-C₆)alkyl)₃, (C₃-C₈)cycloalkyl, (C₂-C₆)alkenyl, (C₂-C₆)alkynyl, aryl, heteraryl, heterocyclyl, halogen, (C₁-C₆)alkoxyl, amino, azido, nitro, cyano, carboxy, sulphonamide, urea, sulphone, acyl, mercapto and hydroxyl. In one embodiment said term "substituted or unsubstituted" refers to a group that may or may not be further substituted with one or more groups selected from (C₁-C₆)alkyl, Si(C₁-C₆)alkyl)₃, (C₁-C₆)cycloalkyl, (C₂-C₆)alkenyl, (C₂-C₆)alkynyl, aryl, heteraryl, heterocyclyl, halogen, (C₁-C₆)alkoxyl, amino, azido, nitro, cyano, carboxy, sulphonamide, urea, sulphone, acyl, mercapto, and the like. In an alternative embodiment "substituted or unsubstituted" refers to a group that may or may not be further substituted with one or more groups selected from (C₂-C₆)alkyl, Si(C₁-C₆)alkyl)₃, (C₃-C₈)cycloalkyl, (C₂-C₆)alkenyl, (C₂-C₆)alkynyl, aryl, heteraryl, heterocyclyl, (C₂-C₆)alkoxyl, amino, azido, cyano, carboxy, sulphonamide, urea, sulphone, acyl and mercapto groups. In another embodiment "substituted or unsubstituted" refers to a group that may or may not be further substituted with one or more groups selected from (C₂-C₆)alkyl, (C₂-C₆)alkenyl, (C₂-C₆)alkoxyl, amino, cyano, carboxy, acyl, mercapto and hydroxyl groups, further preferably (C₂-C₆)alkyl, (C₂-C₆)alkenyl, (C₂-C₆)alkoxyl, amino, cyano, carboxy, acyl and mercapto groups.

A particular preferred embodiment of the present invention refers to a compound of formula (I) according to the present invention, or a pharmaceutically acceptable salt or solvate thereof: 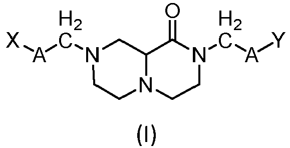 wherein
A is independently selected from the group consisting of: C₁-C₆ alkyl or is absent;
X and Y are independently selected from the group consisting of C₁-C₆ haloalkyl, aromatic carbocyclic 6-membered ring, or an aromatic heterocyclic 5- or 6-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
R⁷ substituent is independently selected from the group consisting of: H, halogen, C₁-C₆ haloalkyl, -O-C₁-C₆ alkyl, -O-C₁-C₂ haloalkyl, cyano and C₁-C₆ alkyl.

An even more preferred embodiment of present invention refers to a compound of formula (I) according to the present invention, or a pharmaceutically acceptable salt thereof: 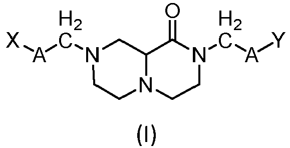 wherein
A is independently selected from the group consisting of: C₁-C₃ alkyl or is absent;
X and Y are independently selected from the group consisting of C₁-C₃ haloalkyl, aromatic carbocyclic 6-membered ring, or an aromatic heterocyclic 5-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
R⁷ substituent is independently selected from the group consisting of: H, F, Cl, Br, C₁-C₃ haloalkyl, -O-C₁-C₆ alkyl, -O-CF3, cyano and C₁-C₆ alkyl.

Another preferred embodiment of present invention refers to compounds of formula (II), or a pharmaceutically acceptable salt thereof, as disclosed herein: 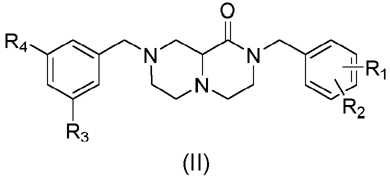 wherein;
R¹ and R² substituent are independently selected from the group consisting of: H, halogen and CF₃;
R³ and R⁴ substituent are independently selected from the group consisting of: halogen and CF₃.

Also within the scope of the present invention are compounds of the invention that are attached to an oligomeric or polymeric framework (e.g., polylysine, dextran, hydroxyethyl starch and a like).

The compounds of formula (I) or formula (II) may also be functionalized to afford compounds having a water-solubility that is enhanced relative to analogous compounds that are not similarly functionalized. Methods of enhancing the water-solubility of organic compounds are known in the art. Such methods include, but are not limited to, functionalizing an organic nucleus with a permanently charged moiety, e.g. quaternary ammonium, or a group that is charged at a physiologically relevant pH, e.g. carboxylic acid, amine. Other methods include, appending to the organic nucleus hydroxyl- or amine-containing groups, e.g. alcohols, polyols, polyethers and the like. Representative examples include, but are not limited to, polylysine, polyethyleneimine, poly(ethyleneglycol) and poly(propyleneglycol). Suitable functionalization chemistries and stragies for these compounds are known in the art. See, for example, Dunn, R.L., *et al., (8).*

Also disclosed herein are metabolites of the compounds of formula (I) or formula (II), according to present invention, which are compounds formed *in vivo* upon administration of the drug.

Metabolites are only covered as long as the structures fall under the definitions of the claims.

Some examples of metabolites include:
(i) where the compound of formula I contains a methyl group, an hydroxymethyl derivative thereof

   (-CH₃→ -CH₂OH);
(ii) where the compound of formula I contains an alkoxy group, a hydroxyl derivative thereof (-OR →-OH);
(iii) where the compound of formula I contains a tertiary amino group,a secondary amino derivative thereof (-NR¹R²→ -NHR¹ or -NHR²);
(iv) where the compound of formula I contains a secondary amino group, a primary amino derivative thereof (-NHR¹→ -NH₂); and
(v) where the compound of formula I contains a phenyl group, a phenol derivative thereof (-Ph→ -PhOH).

The compounds of formula (I) or formula (II) containing one or more asymmetric carbon atoms can exist as two or more stereoisomers. Where a compound of formula (I) or formula (II) contains an alkene or alkenylene group, geometric cis/trans (E/Z) isomers are possible. Where structural isomers are interconvertible via a low energy barrier, tautomeric isomerism ("tautomerism") can occur. This can take the form of proton tautomerism in compounds of formula (I) or formula (II) containing, for example, an imino, keto, or oxime group, or so-called valence tautomerismin compounds which contain an aromatic moiety. It follows that a single compound may exhibit more than one type of isomerism. Thus, the compounds of the present invention also include all stereoisomers, geometric isomers, and tautomeric forms of the compounds of formula (I) or formula (II), as hereinbefore defined, including compounds that exhibit more than one type of isomerism, and mixtures of one or more thereof. Also included are acid or base salts wherein the counter ion is optically active, for example, d-lactate or I-lysine, or racemic, for example, dl-tartrate or dl-arginine.

Cis/trans isomers may be separated by conventional techniques well known to those skilled in the art, for example, chromatography or fractional crystallization.

Where a compound possesses a stable chiral center the compound can be used as a purified enantiomer or diastereomer, or as a mixture of any ratio of stereoisomers. It is however preferred that the mixture comprises at least 70%, 80%, 90%, 95%, 97.5% or 99% of the preferred isomer. Conventional techniques for the preparation/isolation of individual enantiomers include chiral synthesis from a suitable optically pure precursor or enantiomeric enrichment by resolution of the racemate (or the racemate of a salt or derivative) using for example, chiral high pressure liquid chromatography (HPLC), namely using HPLC with an asymmetric resin and a mobile phase. Alternatively, the racemate (or a racemate precursor) may be reacted with a suitable optically active compound, for example, an alcohol, or in the case where the compounds of formula I contains an acidic or basic moiety, a base or acid such as 1-phenylethylamine or tartaric acid. The resulting diastereomeric mixture may be separated by chromatography and/or fractional crystallization, and one or both, of the diastereoisomers converted to the corresponding pure enantiomer(s) by means well known to those skilled in the art.

Also disclosed are pharmaceutically acceptable isotopically labelled compounds of formula (I) or formula (II), as hereinbefore defined, wherein one or more atoms are replaced by atoms having the same atomic number, but an atomic mass or mass number different from the atomic mass or mass number which predominates in nature. Example of isotopes acceptable for inclusion in the compounds of the invention include isotopes of hydrogen such as ²H and ³H, carbons such as ¹¹C, ¹³C and ¹⁴C, chlorine such as ³⁶Cl, fluorine such as ¹⁸F, iodine such as ¹²³I and ¹²⁵I, nitrogen such as ¹³N and ¹⁵N, oxygen such as ¹⁵O, ¹⁷O and ¹⁸O, phosphorus such as ³²P, and sulphur such as ³⁵S. Certain isotopically-labelled compounds of formula I, for example those incorporating a radioactive isotope, are useful in drug and/or substrate tissue distribution studies. The radioactive isotopes tritium, i.e., ³H, and carbon-14, i.e.¹⁴C are particularly useful for this purpose in view of their ease of incorporation and ready means of detection. Substitution with heavier isotopes such as deuterium i.e. ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C and ¹⁸F, ¹⁵O and ¹³N, can be useful in Positron Emission Topography (PET) studies for examining substrate receptor occupancy. Isotopically labelled compounds of formula I can generally be prepared by conventional techniques known to those skilled in the art or by processes analogous to those described in the accompanying Examples and preparations section, using an appropriate isotopically labelled reagent in place of the non-labelled reagent previously employed.

The compounds of formula (I) or formula (II) according to the present invention activate human ClpP and in doing so cause an imbalance in the cell's proteome. In the case of human cancer cells that overexpress HsClpP, this imbalance leads to mitochondria stress and eventually apoptosis.

The compounds of formula (I) or formula (II) according to present invention activate HsClpP, and as a consequence of this activation, our experimental evidence presented in this application indicates that they possess anticancer properties.

The compounds of formula (II) according to present invention also can activate SaClpP, and as a consequence of this activation, our experimental evidence presented in this application indicates that they possess antibacterial properties.

Accordingly, one embodiment of the present invention refers to a pharmaceutical composition comprising at least one compound of formula (I) or formula (II), according to the present invention, or a pharmaceutically acceptable salt thereof, and at least one pharmaceutically acceptable excipient.

Another embodiment of present invention refers to an antibody-drug conjugate comprising a compound of formula (I) or formula (II) and a human or humanized monoclonal or polyclonal antibody.

For the purposes of present invention, the term "antibody-drug conjugate" refers to the coupling of a compound, in particular a compound of formula (I) or formula (II) or a pharmaceutically acceptable salt thereof, according to present invention, with a human or humanized monoclonal or polyclonal antibody such as IgG1, IgG2 or IgG4. In an embodiment the compound of formula (I) or formula (II) and the human or humanized monoclonal or polyclonal antibody are linked by a cleavable or a non-cleavable linker, depending on whether the bond between the antibody and the compound of formula (I) or formula (II) may or may not be degraded or broken down by enzymes.

Yet another embodiment of present invention refers to a pharmaceutical composition comprising at least one antibody-drug conjugate comprising a compound of formula (I) or formula (II), according to the present invention, and at least one pharmaceutically acceptable excipient.

Additionally, present invention also refers to a compound of formula (I) or formula (II), or a pharmaceutically acceptable salt thereof, or to an antibody-drug conjugate comprising the same, according to the present invention, for use as a medicament. In particular, one embodiment of present invention refers to a compound of formula (I) or formula (II), or a pharmaceutically acceptable salt thereof, or to an antibody-drug conjugate comprising the same, according to the present invention, for use in the prevention and/or treatment of a disease or condition responsive or ameliorated by activation of caseinolytic protease P (ClpP). Preferably, present invention refers to a compound of formula (I) or formula (II), or a pharmaceutically acceptable salt thereof, or to an antibody-drug conjugate comprising the same, according to the present invention, for use in the prevention and/or treatment of cancer.

Another embodiment disclosed in present invention refers to a pharmaceutical composition comprising at least one compound of formula (I) or formula (II), or a pharmaceutically acceptable salt thereof, or an antibody-drug conjugate comprising the same, according to the present invention, and at least one pharmaceutically acceptable excipient, for use as a medicament and, in particular, for use in the prevention and/or treatment of a disease or condition responsive or ameliorated by activation of caseinolytic protease P (ClpP). Preferably, present invention refers to a pharmaceutical composition comprising at least one compound of formula (I) or formula (II), or a pharmaceutically acceptable salt thereof, or an antibody-drug conjugate comprising the same, according to the present invention, for use in the prevention and/or treatment of cancer.

Also described is the use of a compound of formula (I) or formula (II), or a pharmaceutically acceptable salt thereof, or an antibody-drug conjugate or a pharmaceutical composition comprising the same, according to the present invention, in the manufacturing of a medicament for preventing /and/or treating a disease or condition responsive to activation of caseinolytic protease P (ClpP) and, preferably, for prevention and/or treatment of cancer.

Additionally, described is a method for preventing and/or treating a disease or condition responsive or ameliorated by activation of caseinolytic protease P (ClpP), wherein said method comprises administering an effective amount of at least one compound of formula (I) or formula (II), or a pharmaceutically acceptable salt thereof, or an antibody-drug conjugate or a pharmaceutical composition comprising the same, according to the present invention, to a subject in need thereof and, preferably, for prevention and/or treatment of cancer.

For the purposes of present invention, the term "disease or condition responsive or ameliorated by activation of caseinolytic protease P (ClpP)" refers to a disease or pathological disorder or condition which may be treated with activators of caseinolytic protease P (ClpP), i.e. a disease or pathological disorder or condition the treatment of which benefits or is ameliorated by activation of caseinolytic protease P (ClpP) and, preferably, for prevention and/or treatment of cancer.

For the purposes of present invention said condition responsive or ameliorated by activation of human caseinolytic protease P (HsClpP) is selected from the group consisting of a hyperproliferative disease, or cancer.

Preferably, said condition responsive or ameliorated by activation of human caseinolytic protease P (HsClpP) is a cancer. More preferably, said cancer has elevated protein expression levels of caseinolytic protease P.

In a preferred embodiment, said cancer, is selected from the group consisting of: sarcoma, breast cancer, prostate cancer, bone cancer, head and neck cancer, brain tumour, glioma, glioblastoma, colorectal cancer, lung cancer, pancreatic cancer, cervical cancer, ovarian cancer, gastric cancer, renal cell carcinoma, melanoma, endometrial cancer, hepatocellular carcinoma, chronic myelogenous leukaemia, acute myelogenous leukaemia, cutaneous T-cell lymphoma, Hodgkin's disease, anaplastic large-cell lymphoma and Burkitt's lymphoma.

In one embodiment, the pharmaceutical compositions disclosed comprise at least one additional active compound or therapeutic ingredient. Said additional active compound or therapeutic ingredient provides additive or synergistic biological activities. For the purposes of present description, the terms "active compound" or "therapeutic ingredient" should be taken as synonyms and mean a chemical or biological entity which exerts therapeutic effects when administered to human or animal beings. Said active compound or therapeutic ingredient exerts therapeutic effects when administered to human or animal beings, and it may be a cell therapy, a small molecule therapy, immunotherapy, radiotherapy, or an antibiotic, among others.

In a preferred embodiment said additional active compound or therapeutic agent is selected from the group consisting of a CAR-T cell therapy, a CAR-NK cell therapy, an antibody, a HIF pathway inhibitor, and a chemotherapeutic agent. Said additional active compound or additional therapy may be administered at the same time, prior to, subsequently to, or at a different moment than the compound of formula (I) for use according to present invention. Preferably, said chemotherapeutic agent is selected from the group consisting of platinum-based antineoplastic agents, anti-mitotic chemotherapeutic agents, a poly adenosine diphosphate ribose polymerase (PARP) inhibitor, type I topoisomerase inhibitors, type II topoisomerase inhibitors, epothilones, cycloskeletal disruptors, alkylating agents, epothilones, histone deacetylase inhibitors, kinase inhibitors, antifolates, kinase inhibitors, peptide antibiotics, retinoids, vinca alkaloids and thymidylate synthase inhibitors. More preferably, the chemotherapeutic agent is selected from the group consisting of cyclophosphamide, ifosfamide, busulfan, temozolomide, mechlorethamine, chlorambucil, melphalan, dacarbazine, daunorubicin, doxorubicin, daunorubicin, epirubicin, idarubicin, mitoxantrone, valrubicin, paclitaxel, docetaxel, abraxane, taxotere, epothilone, vorinostat, romidepsin, irinotecan, topotecan, camptothecin, exatecan, lurtotecan, etoposide, teniposide, tafluposide, bortezomib, erlotinib, gefitinib, imatinib, vemurafenib, vismodegib, azacitadine, azathioprine, capecitabine, cytarabine, cladribine, fludarabine, doxifluridine, fluorouracil, gemcitabine, hydroxyurea, mercaptopurine, methotrexate, pemetrexed, tioguanine, bleomycin, actinomycin, carboplatin, cisplatin, oxaliplatin, tretinoin, alitretinoin, bexarotene, vinblastine, vincristine, vindesine and vinorelbine.

In another preferred embodiment said additional active ingredient is an antibiotic selected from the group consisting of penicillin G, naficillin, oxacillin, ampicillin, amoxicillin, cefazolin, cephalexin, cephotetan, cefoxitin, ceftriaxone, ceftazidime, cefepime, ertapenem, imipenem, meropenem, ciproflaxacin, levofloxacin, moxifloxacin, amikacin, tobramycin, gentamicin, clindamycin, azithromycin, doxyclycine, vancomycin, rifaximin, linezolid, colistin, rifampicin, and isoniazid.

In yet another preferred embodiment said additional active ingredient is a human or humanized monoclonal or polyclonal antibody.

Preferably said antibody is selected mogamulizumab, blinatumomab, ibritumomab, obinutuzumab, ofatumumab, rituximab, tositumomab, inotuzumab, moxetumomab, brentuximab, gemtuzumab, daratumumab, isatuximab, alemtuzumab, polatuzumab, cetuximab, necitumumab, nimotuzumab, panitumumab, catumaxomab, burosumab, dinutuximab, pertuzumab, trastuzumab, ertumaxomab, mepolizumab, siltuximab, enfortumab, etaracizumab, racotumomab, bevacizumab, denosumab, elotuzumab, olaratumab, ramucirumab, bermekimab, labetuzumab, pemtumomab, tacatuzumab, pembrolizumab, nivolumab, cemiplimab, atezolizumab, avelumab, durvalumab, ipilimumab or any other immunotherapeutic agent.

The compounds of formula (I) or formula (II) and pharmaceutical compositions comprising the same, for use according to present invention may also be administered together, prior to, or subsequently to an additional therapy. Preferably said additional therapy is radiotherapy, immunotherapy, chemotherapy or an antibacterial treatment.

In a preferred embodiment, said compound of formula (I) or formula (II), or said pharmaceutical composition for use according to present invention is administered together with, prior to or subsequently to a radiotherapeutic treatment, a chemotherapeutic treatment, an immunotherapeutic treatment, or an antibacterial treatment.

Additionally, the compounds of formula (I) or formula (II) or pharmaceutically salts and solvates thereof, for use according to present invention may also be administered as an antibody-drug conjugate or administered as part of an antibody-drug conjugate, i.e., administered as an antibody-drug conjugate comprising said compounds of formula (I) or pharmaceutically salts and solvates thereof and a human or humanized monoclonal or polyclonal antibody.

Another embodiment of present invention refers to a compound of formula (II), or a pharmaceutically acceptable salt thereof, or to an antibody-drug conjugate comprising the same, according to the present invention, for use as a medicament. In particular, one embodiment of present invention refers to a compound of formula (II), or a pharmaceutically acceptable salt thereof, or to an antibody-drug conjugate comprising the same, according to the present invention, for use in the prevention and/or treatment of a disease or condition responsive or ameliorated by activation of bacterial caseinolytic protease P such as *Staphylococcus aureus* caseinolytic protease P (SaClpP). Preferably, present invention refers to a compound formula (II), or a pharmaceutically acceptable salt thereof, or to an antibody-drug conjugate comprising the same, according to the present invention, for use in the prevention and/or treatment of bacterial infection.

Additionally, present invention also refers to a pharmaceutical composition comprising at least one compound of formula (II), or a pharmaceutically acceptable salt thereof, or an antibody-drug conjugate comprising the same, according to the present invention, and at least one pharmaceutically acceptable excipient, for use as a medicament and, in particular, for use in the prevention and/or treatment of a disease or condition responsive or ameliorated by activation of bacterial caseinolytic protease P, such as *Staphylococcus aureus* caseinolytic protease P (SaClpP). Preferably, present invention refers to a pharmaceutical composition comprising at least one compound of formula (II), or a pharmaceutically acceptable salt thereof, or an antibody-drug conjugate comprising the same, according to the present invention, for use in the prevention and/or treatment of bacterial infection.

Also described is the use of a compound of formula (II), or a pharmaceutically acceptable salt thereof, or an antibody-drug conjugate or a pharmaceutical composition comprising the same, according to the present invention, in the prevention and/or treatment of bacterial infection caused by *Staphylococcus aureus.*

For the purposes of present invention, the term "disease or condition responsive or ameliorated by activation of bacterial caseinolytic protease P, such as *Staphylococcus aureus* caseinolytic protease P (SaClpP)" refers to a disease or pathological disorder or condition which may be treated with activators of *Staphylococcus aureus* caseinolytic protease P (SaClpP), i.e. a disease or pathological disorder or condition the treatment of which benefits or is ameliorated by activation of *Staphylococcus aureus* caseinolytic protease P (SaClpP) and, preferably, for prevention and/or treatment of bacterial infection.

For the purposes of present invention said condition responsive or ameliorated by activation of *Staphylococcus aureus* caseinolytic protease P (SaClpP) is a bacterial infection, preferably *Staphylococcus aureus* infection.

The compounds of the present invention can be prepared and administered in a wide variety of oral, parenteral, and topical dosage forms. Thus, the compounds of the present invention can be administered by injection, that is, intravenously, intramuscularly, intracutaneously, subcutaneously, intraduodenally, or intraperitoneally. Also, the compounds described herein can be administered by inhalation, for example, intranasally. Additionally, the compounds of the present invention can be administered transdermal.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration. A solid carrier can be one or more substances, which may also act as diluents, flavouring agents, binders, preservatives, tablet disintegrating agents, or an encapsulating material.

In powders and tablets, preferably containing from 5% to 80% of the active compound, suitable carriers are magnesium carbonate, magnesium stearate, talc, sugar, lactose, pectin, dextrin, starch, gelatine, tragacanth, methylcellulose, sodium carboxymethylcellulose, a low melting wax, cocoa butter, hydroxypropylmethylcellulose, polyethyleneglycol, creamaphor, and the like.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized moulds, allowed to cool, and thereby to solidify.

Liquid preparations include solutions, suspensions, and emulsions, for example, water or water/propylene glycol solutions. For peritoneal injection, liquid preparations can be formulated in solution in aqueous polyethylene glycol solution.

Aqueous solutions suitable for oral use can be prepared by dissolving the active component in water and adding suitable colorants, flavours, stabilizers, and thickening agents as desired. Aqueous suspensions suitable for oral use can be prepared by dispersing the finely divided active component in water with viscous material, such as natural or synthetic gums, resins, methylcellulose, cremaphor, sodium carboxymethylcellulose, and other well-known suspending agents.

Thus, a pharmaceutically acceptable carrier may comprise magnesium carbonate, magnesium stearate, talc, sugar, lactose, pectin, dextrin, starch, gelatine, tragacanth, methylcellulose, sodium carboxymethylcellulose, low melting fatty acid glycerides, cocoa butter, water, propylene glycol, natural or synthetic gums, resins, cremaphor, methylcellulose, and sodium carboxymethylcellulose.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 10000 mg, more typically 1.0 mg to 1000 mg, most typically 10 mg to 500 mg, according to the particular application, and the potency of the active component.

The compounds of the present invention, or their pharmaceutically acceptable salts and solvates or prodrugs, (prodrugs do not form part of the invention) are administered in a therapeutically effective amount which will vary depending upon a variety of factors including the activity of the specific compound employed, the metabolic stability and length of action of the compound, age, body weight, general health, sex, diet, mode and time of administration, rate of excretion, drug combination effects, the severity of the particular disease, and the patients side effect profile towards the compound.

Determination of a therapeutically effective amount of a compound of the invention is well within the capabilities of those skilled in the art.

Patient doses for oral administration of the compounds described herein, which is the preferred mode of administration for prophylaxis treatment of an exemplary disease such as cancer, typically range from about 0.1 mg/day to about 10000 mg/day, more typically from about 1 mg/day to about 1000 mg/day, and most typically 1 mg/day to 500 mg/day. Stated in terms of patient body weight, typical dosages range from about 0.01 to about 150 mg/kg/day, more typically from about 0.1 to about 15 mg/kg/day, and most typically from about 0.5 to about 10 mg/kg/day.

For other modes of administration, dosage amount and interval, can be adjusted individually to provide plasma levels of the administered compound, that are effective for the particular clinical indication being treated.

The method by which the compounds of the present invention are prepared is not critical; compounds of the invention, including salts and solvates and prodrugs thereof, can be prepared using known organic synthesis techniques and can be synthesized according to any of numerous possible synthetic routes.

The reactions for preparing compounds of the invention can be carried out in suitable solvents which can be readily selected by one skilled in the art of organic synthesis. A given reaction can be carried out in one solvent or a mixture of more than one solvent and at temperatures that can range from the solvent's freezing temperature to the solvent's boiling temperature. In cases where sealed tube assisted heating is used then the solvent temperature may exceed the boiling temperature of the solvent.

Preparation of compounds of the invention can involve protection and deprotection of various chemical groups. The need for protection and deprotection, and the selection of appropriate protecting groups, can be readily determined by one skilled in the art. The chemistry of protecting groups can be found, for example, in Greene, T.W. et al., (9) which is incorporated herein by reference in its entirety. Reactions can be monitored according to any suitable method known in the art. For example, product formation can be monitored by chromatographic methods such as thin layer chromatography (TLC) or high-performance liquid chromatography (HPLC), or by spectroscopic means, such as nuclear magnetic resonance spectroscopy (e.g., ¹H, ¹³C or ¹⁹F), infrared spectroscopy, spectrophotometry (e.g., UV-visible) or mass spectroscopy.

In an exemplary reaction pathway, compounds of the invention can be prepared following the methods outlined in the general synthetic scheme A:

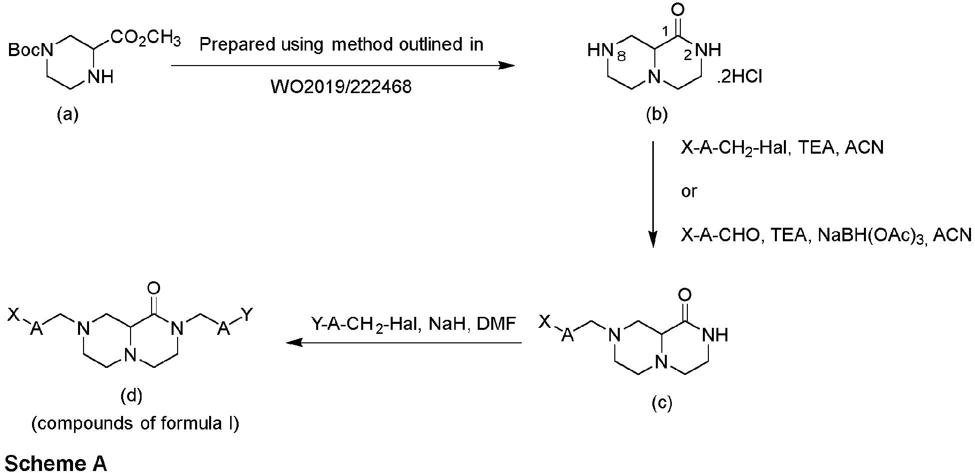

Boc protected piperazine-2-carboxylate (a), which are either commercially available or readily prepared using published chemistry, are transformed into the bicyclic amine (b) via a three-step process outlined in the patent application WO2019/222468. Preferential alkylation of the N8 nitrogen of (b) by either a typical nucleophilic substitution reaction or a classical reductive animation reaction generates intermediate (c). N-alkylation of the amide N2 nitrogen under basic conditions provides compounds (d) of formula (I).

Enantiomeric versions of compounds of formula (I) were prepared using the synthetic scheme outlined in scheme B. The commercially available enantiomerically enriched (R) or (S) versions of compound (a) (e.e > 98%) were transformed into the Boc-protected enantiomeric core compound (f) following a two-step procedure outlined in the patent application WO2023/016521. Compound (f) could be deprotected to generate the chiral versions of compound (b) in scheme A, which could be transformed into the desired final products are previously described above. Alternatively, compound (f) could initially be alkylated at the amide nitrogen, Boc-deprotected and alkylated at the free amine site to generate the compounds of formula (I).

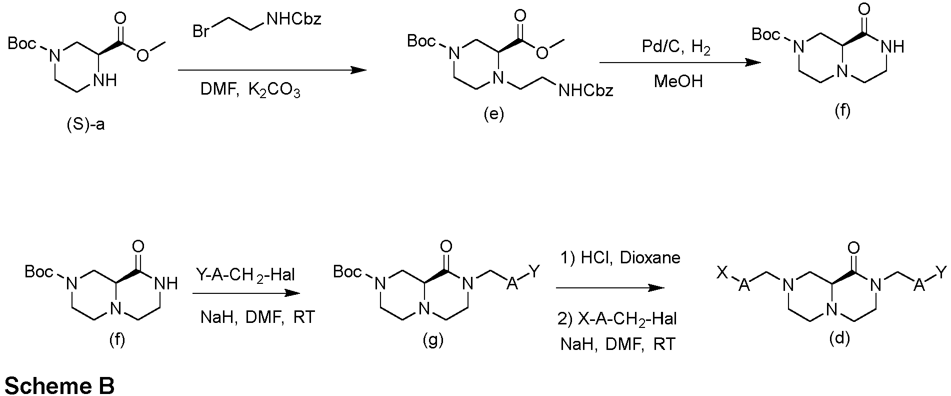

### EXAMPLES

The following examples are offered to illustrate, but not to limit, the claimed invention.

According to the general synthetic schemes A, the following compounds of formula (I), included in the following **table 1** (ID# 1 to 38), below, were prepared:

| **ID** | **Structure** | **ID** | **Structure** |
|---|---|---|---|
| 1 | 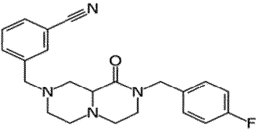 | 9 | 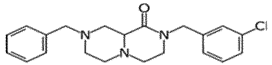 |
| 2 | 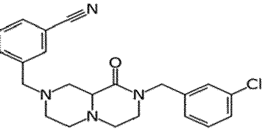 | 10 | 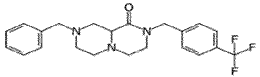 |
| 3 | 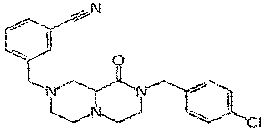 | 11 | 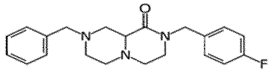 |
| 4 | 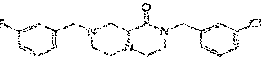 | 12 | 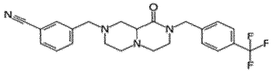 |
| 5 | 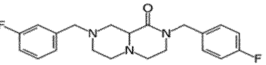 | 13 | 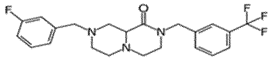 |
| 6 | 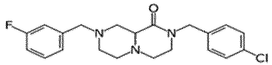 | 14 | 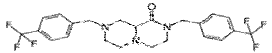 |
| 7 | 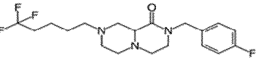 | 15 | 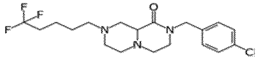 |
| 8 | 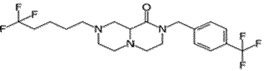 | 16 | 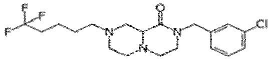 |
| 17 | 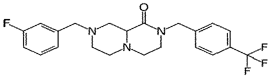 | 26 | 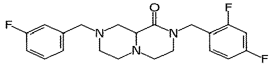 |
| 18 | 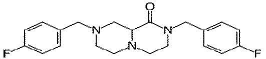 | 27 | 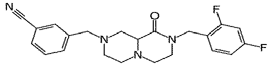 |
| 19 | 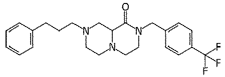 | 28 | 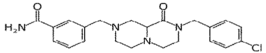 |
| 20 | 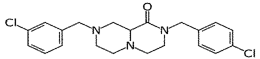 | 29 | 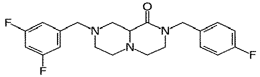 |
| 21 | 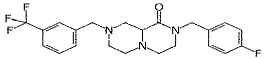 | 30 | 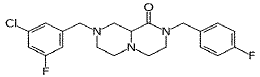 |
| 22 | 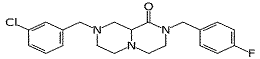 | 31 | 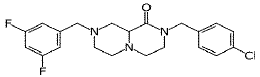 |
| 23 | 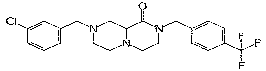 | 32 | 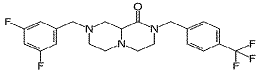 |
| 24 | 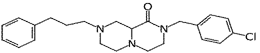 | 33 | 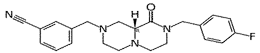 |
| 25 | 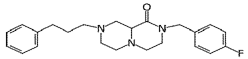 | 34 | 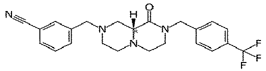 |
| 35 | 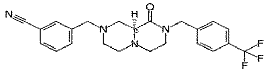 | | |
| 36 | 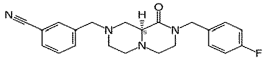 | | |
| 37 | 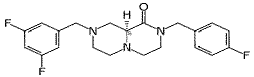 | | |
| 38 | 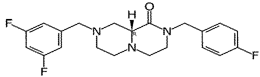 | | |

In the examples below, unless otherwise stated, temperatures are given in degrees Celsius (°C); operations were carried out at room, or ambient temperature (typically a range from 15-25°C); evaporation of solvent was carried out using a rotary evaporator under reduced pressure (typically 4-30mmHg/ with a bath temperature of up to 60°C; and the following conventional abbreviations are also used: mp (melting point), L (liter(s)), mL (millitres), mmol (millimoles), g (grams), mg (milligrams), min (minutes), and h (hours).

### EXAMPLE 1: Synthesis of N8-substitutedhexahydro-2H-pyrazino[1,2-a]pyrazin-1(6H)-ones (b) according to general scheme A.

### 1.1. General procedure for the alkylation of compounds (a) to prepare compounds (b).

Triethylamine (3 eq) was added to a suspension of exahydro-2H-pyrazino[1,2-a]pyrazin-1(6H)-one dihydrochloride (b1, 1 eq) in acetonitrile (10 ml /mmol of b1) at rt. A benzyl bromide derivative (1.1 eq) was then added dropwise and the resulting mixture stirred at rt for 3h. The solvent was removed by rotary evaporation and ethyl acetate (10 ml) added. The organics were washed sequentially with water and brine, then dried (MgSO₄) and filtered. The solvent was removed under reduced pressure to afford an oil which was used without further purification.

### 1.1.1 Preparation of 8-(3-fluorobenzyl)hexahydro-2H-pyrazino[1,2-a]pyrazin-1(6H)-one (b1)

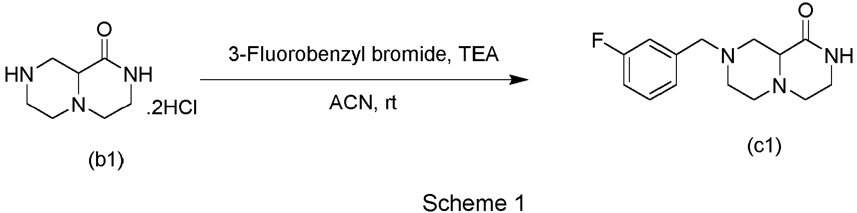

Compound (c1) was prepared using the general procedure described in part 1.1 using 3-fluorobenzyl bromide as the benzyl bromide derivative. Product was a colourless oil. Yield = 82 %

The following intermediates (c2-c11) were prepared using the same synthetic method described in example 1.1. Yields ranged from 67-85%

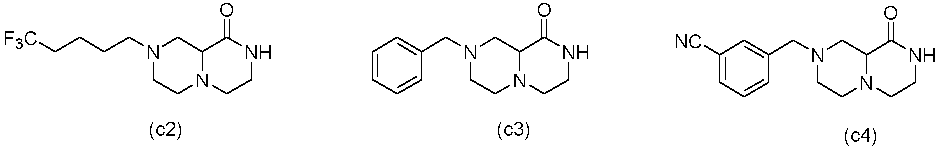

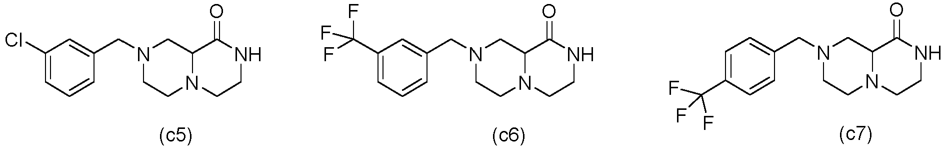

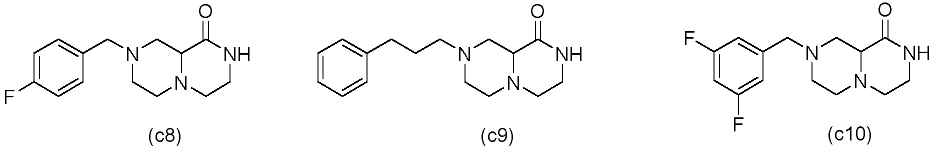

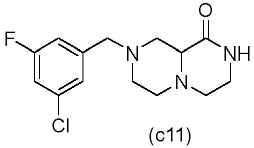

### EXAMPLE 2: Synthesis of compounds of formula (I) (n = 1) according to general scheme A.

### 2.1. General method for the preparation of N2, N8 bis-substituted hexahydro-2H-pyrazino[1,2-a]pyrazin-1(6H)-one (d)

NaH (60% in mineral oil, 1.5 eq) was added to a stirring solution of intermediate (c) (1 eq) in anhydrous DMF (0.2M) under Argon at room temperature. After 15 minutes, the desired alkylating reagent was added (1 eq). The reaction mixture was allowed to stir for 3-6 hours at room temperature. Water was added and the solvents removed by lyophilization. DCM was added to the residue and the organics were filtered to remove the inorganic salts.

Concentration of the organics followed by purification by column chromatography (DCM/MeOH; 98.:2) and removal of the solvents afforded the desired product, generally as a colourless viscous oil, or a white solid.

### 2.1.1 Preparation of 8-(3-fluorobenzyl)-2-(3-(trifluoromethyl)benzyl)hexahydro-2H-pyrazino[1,2-a]pyrazin-1(6H)-one (13)

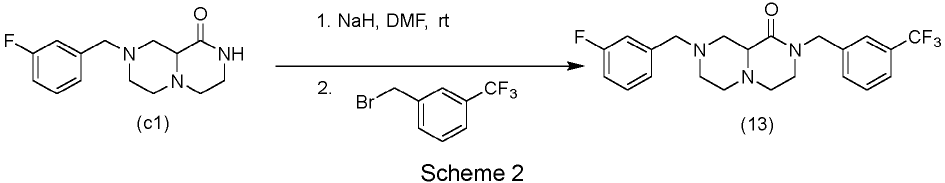

Compound (13) was prepared using the general procedure described in part 2.1, using 3-(trifluoromethyl)benzyl bromide as the alkylating agent. Product was an off white solid. Yield = 37 %

Using the same synthetic method described in example 2.1, the following compounds of formula (I) were prepared:

| Reactant (c) | Alkylating reagent | Compound ID # |
|---|---|---|
| c1 | 4-Fluorobenzyl bromide | 5 |
| c1 | 4-Chlorobenzyl bromide | 6 |
| c1 | 3-Chlorobenzyl bromide | 4 |
| c1 | 4-(Trifluoromethyl)benzyl bromide | 17 |
| c1 | 2,4-difluorobenzyl bromide | 26 |
| c2 | 4-fluorobenzyl bromide | 7 |
| c2 | 4-(Trifluoromethyl)benzyl bromide | 8 |
| c2 | 4-Chlorobenzyl bromide | 15 |
| c2 | 3-Chlorobenzyl bromide | 16 |
| c3 | 3-Chlorobenzyl bromide | 9 |
| c3 | 4-fluorobenzyl bromide | 11 |
| c3 | 4-(Trifluoromethyl)benzyl bromide | 10 |
| c4 | 4-(Trifluoromethyl)benzyl bromide | 12 |
| c4 | 4-fluorobenzyl bromide | 1 |
| c4 | 3-Chlorobenzyl bromide | 2 |
| c4 | 4-Chlorobenzyl bromide | 3 |
| c4 | 2,4-difluorobenzyl bromide | 27 |
| c5 | 4-Chlorobenzyl bromide | 20 |
| c5 | 4-fluorobenzyl bromide | 22 |
| c5 | 4-(Trifluoromethyl)benzyl bromide | 23 |
| c6 | 4-fluorobenzyl bromide | 21 |
| c7 | 4-(Trifluoromethyl)benzyl bromide | 14 |
| c8 | 4-fluorobenzyl bromide | 18 |
| c9 | 4-(Trifluoromethyl)benzyl bromide | 19 |
| c9 | 4-Chlorobenzyl bromide | 24 |
| c9 | 4-fluorobenzyl bromide | 25 |
| c10 | 4-fluorobenzyl bromide | 29 |
| c10 | 4-Chlorobenzyl bromide | 31 |
| c10 | 4-(Trifluoromethyl)benzyl bromide | 32 |
| c11 | 4-fluorobenzyl bromide | 30 |

### Examples of spectral data for compounds of formula (I):

Compound (8. 2HCl): ¹H NMR (d₆-DMSO, 500 MHz) : δ 7.78 (d, 2H, *J* = 7.8 Hz), 7.53 (d, 2H, *J* = 8.0 Hz), 4.70 (d, 1H, *J =* 14.7 Hz), 4.65 (d, 1H, *J =* 15.3 Hz), 3.88 (d, 1H, *J =* 11.4 Hz), 3.55 (d, 1H, *J =* 11.7 Hz), 3.49-3.41 (m, 2H), 3.40-3.34 (m, 1H), 3.27 (d, 2H, *J* = 9.8 Hz), 3.20-3.08 (m, 2H), 3.07-2.95 (m, 2H), 2.68 (d, 2H, *J =* 12.6 Hz), 2.43-2.32 (m, 2H), 1.91-1.81 (m, 2H), 1.63-1.55 (m, 2H).

Compound (5): ¹H NMR (CDCl₃, 500 MHz) : δ 7.28-7.19 (m, 3H), 7.10-7.05 (m, 2H), 7.03-6.97 (m, 2H), 6.96-6.91 (m, 1H), 4.66 (d, 1H, *J* = 14.6 Hz), 4.37 (d, 1H, *J* = 14.6 Hz), 3.62 (d, 1H, *J* = 13.3 Hz), 3.52 (d, 1H, *J =* 13.4 Hz), 3.50-3.42 (m, 2H), 3.06 (dd, 1H, *J =* 11.5, 4.0 Hz), 2.91-2.85 (m, 2H), 2.82-2.73 (m, 2H), 2.52 (dt, 1H, *J* = 11.9, 4.3 Hz), 2.38 (dt, 1H, *J* = 11.2, 2.7 Hz), 2.22 (dt, 1H, *J* = 11.0, 2.5 Hz), 2.14-2.09 (m, 1H).

Compound (6): ¹H NMR (CDCl₃, 500 MHz) : δ 7.23-7.16 (m, 3H), 7.10 (d, 2H, J = 8.3 Hz), 7.02-6.97 (m, 2H), 6.88-6.83 (m, 1H), 4.59 (d, 1H, *J =* 14.7 Hz), 4.29 (d, 1H, *J =* 14.7 Hz), 3.55 (d, 1H, *J =* 13.4 Hz), 3.44 (d, 1H, *J* = 13.4 Hz), 3.42-3.35 (m, 2H), 2.98 (dd, 1H, *J* = 11.5, 3.9 Hz), 2.83-2.78 (m, 2H), 2.74-2.66 (m, 2H), 2.45 (dt, 1H, *J =* 12.0, 4.3 Hz), 2.31 (dt, 1H, *J* = 11.2, 2.7 Hz), 2.15 (dt, 1H, *J =*11.0*,* 2.5 Hz), 2.07-2.00 (m, 1H).

Compound (3): ¹H NMR (CDCl₃, 500 MHz) : δ 7.66 (s, 1H), 7.57-7.52 (m, 2H), 7.43-7.39 (m, 1H), 7.29 (d, 2H, *J =* 8.3 Hz), 7.18 (d, 2H, *J* = 8.4 Hz), 4.65 (d, 1H, *J* = 14.7 Hz), 4.36 (d, 1H, *J* = 14.7 Hz), 3.63 (d, 1H, *J =* 13.7 Hz), 3.56 (d, 1H, *J* = 13.7 Hz), 3.51-3.40 (m, 2H), 3.06 (dd, 1H, *J* = 11.4, 4.0 Hz), 2.92-2.84 (m, 2H), 2.82 (d, 1H, *J* = 10.8 Hz), 2.73 (d, 1H, *J* = 10.9 Hz), 2.54 (dt, 1H, *J =* 11.9, 4.3 Hz), 2.39 (dt, 1H, *J =* 11.2, 2.7 Hz), 2.26 (dt, 1H, *J =* 11.1, 2.6 Hz), 2.14-2.09 (m, 1H).

Compound (2): ¹H NMR (CDCl₃, 500 MHz) : δ 7.66 (s, 1H), 7.58-7.53 (m, 2H), 7.44-7.39 (m, 1H), 7.27-7.21 (m, 3H), 7.15-7.11 (m, 1H), 4.68 (d, 1H, *J =* 14.8 Hz), 4.37 (d, 1H, *J =* 14.8 Hz), 3.64 (d, 1H, *J =* 13.7 Hz), 3.56 (d, 1H, *J =* 13.7 Hz), 3.52-3.41 (m, 2H), 3.08 (dd, 1H, *J =* 11.5, 4.0 Hz), 2.92-2.84 (m, 2H), 2.82 (d, 1H, *J =* 10.8 Hz), 2.73 (dd, 1H, *J =* 10.8, 1.5 Hz), 2.56 (dt, 1H, *J =* 12.0, 4.3 Hz), 2.40 (dt, 1H, *J =* 11.1, 2.7 Hz), 2.26 (dt, 1H, *J =* 11.1, 2.6 Hz), 2.16-2.10 (m, 1H).

Compound (14): ¹H NMR (CDCl₃, 500 MHz) : δ 7.66-7.55 (m, 4H), 7.45 (d, 2H, *J =* 7.9 Hz), 7.35 (d, 2H, *J =* 7.9 Hz), 4.73 (d, 1H, *J =* 14.9 Hz), 4.48 (d, 1H, *J =* 14.9 Hz), 3.68 (d, 1H, *J =* 13.5 Hz), 3.59 (d, 1H, *J* = 13.5 Hz), 3.52-3.44 (m, 2H), 3.08 (dd, 1H, *J* = 11.5, 3.8 Hz), 2.93-2.88 (m, 2H), 2.83-2.73 (m, 2H), 2.56 (dt, 1H, *J =* 12.0, 4.2 Hz), 2.40 (dt, 1H, *J =* 11.1, 2.4 Hz), 2.25 (dt, 1H, *J =* 11.1, 2.3 Hz), 2.17-2.11 (m, 1H).

Compound (10): ¹H NMR (CDCl₃, 500 MHz) : δ 7.58 (d, 2H, *J =* 8.0 Hz), 7.38-7.23 (m, 7H), 4.73 (d, 1H, *J = 15.1* Hz), 4.47 (d, 1H, *J =* 15.1 Hz), 3.67 (d, 1H, *J =* 12.6 Hz), 3.54 (d, 1H, *J =* 13.2 Hz), 3.52-3.45 (m, 2H), 3.07 (dd, 1H, *J* = 11.5, 3.5 Hz), 2.94-2.86 (m, 2H), 2.83-2.76 (m, 2H), 2.55 (dt, 1H, *J* = 11.9, 4.0 Hz), 2.45-2.36 (m, 1H), 2.26-2.19 (m, 1H), 2.16-2.10 (m, 1H).

Compound (9): ¹H NMR (CDCl₃, 500 MHz): δ 7.33-7.29 (m, 4H), 7.27-7.22 (m, 4H), 7.14-7.11 (m, 1H), 4.69 (d, 1H, *J =* 14.7 Hz), 4.35 (d, 1H, *J =* 14.7 Hz), 3.66 (d, 1H, *J =* 13.1 Hz), 3.53 (d, 1H, *J =* 12.7 Hz), 3.51-3.43 (m, 2H), 3.06 (dd, 1 H, *J* = 11.5, 3.6 Hz), 2.93-2.86 (m, 2H), 2.82-2.76 (m, 2H), 2.54 (dt, 1H, *J* = 11.8, 4.0 Hz), 2.43-2.36 (m, 1H), 2.25-2.18 (m, 1H), 2.16-2.10 (m, 1H).

Compound (12. 2HCl): ¹H NMR (d₆-DMSO, 500 MHz) : δ 8.18-8.04 (m, 1H), 8.00-7.90 (m, 2H), 7.73 (d, 2H, *J* = 7.9 Hz), 7.70 (t, 1H, *J* = 7.8 Hz), 7.47 (d, 2H, *J* = 7.9 Hz), 4.61 (s, 2H), 4.53-4.32 (m, 2H), 3.84-3.59 (m, 4H), 3.50-3.27 (m, 3H), 3.27-3.18 (m, 1H), 3.15-2.93 (m, 4H).

Compound (11. 2HCl): ¹H NMR (d₆-DMSO, 500 MHz) : δ 7.67-7.62 (m,2H), 7.50-7.45 (m, 3H), 7.31-7.26 (m, 2H), 7.17 (t, 2H, *J* = 8.8 Hz), 4.53 (d, 1H, J = 14.7 Hz), 4.46 (d, 1H, J = 14.8 Hz), 4.38 (s, 2H), 3.75-3.64 (m, 1H), 3.59-3.42 (m, 1H), 3.41-3.30 (m, 2H), 3.23-315 (m, 1H), 3.14-2.94 (m, 4H), 2.84-2.71 (m, 1H), 2.70-2.58 (m, 1H).

Compound (7. 2HCl): ¹H NMR (d₆-DMSO, 500 MHz) : δ 7.33-7.27 (m, 2H), 7.18 (t, 2H, J = 8.8 Hz), 4.57 (d, 1H, *J* = 14.6 Hz), 4.47 (d, 1H, *J* = 14.7 Hz), 3.83 (d, 1H, J = 11.8 Hz), 3.56-3.45 (m, 2H), 3.44-3.30 (m, 1H), 3.26-3.16 (m, 3H), 3.15-3.05 (m, 2H), 3.04-2.91 (m, 2H), 2.80-2.56 (m, 2H), 2.40-2.27 (m, 2H), 1.87-1.76 (m, 2H), 1.53 (p, 2H, *J* = 7.5 Hz).

Compound (4. 2HCl): ¹H NMR (d₆-DMSO, 500 MHz) : δ 7.66-7.57 (m, 1H), 7.56-7.50 (m, 1H), 7.49-7.44 (m, 1H), 7.42-7.33 (m, 3H), 7.32-7.29 (m, 1H), 7.25-7.20 (m, 1H), 4.58-4.47 (m, 2H), 4.45-4.34 (m, 2H), 3.75-3.65 (m, 1H), 3.48-3.36 (m, 1H), 3.35-3.28 (m, 1H), 3.27-2.93 (m, 6H), 2.90-2.63 (m, 2H).

Compound (1. 2HCl): ¹H NMR (d₆-DMSO, 500 MHz) : δ 8.15 (brs, 1H), 7.99 (d, 1H, J = 7.3 Hz), 7.95 (d, 1H, *J* = 7.8 Hz), 7.69 (t, 1H, *J* = 7.8 Hz), 7.30 (dd, 2H, *J* = 8.2, 6.0 Hz), 7.17 (t, 2H, *J* = 8.9 Hz), 4.53 (d, 1H, *J =* 15.0 Hz), 4.49 (d, 1H, *J* = 15.0 Hz), 4.46-4.34 (m, 2H), 3.74-3.49 (m, 2H), 3.46-3.36 (m, 1H), 3.34-3.26 (m, 1H), 3.26-2.97 (m, 5H), 2.87-2.65 (m, 2H).

Compound (13): ¹H NMR (CDCl₃, 500 MHz) : δ 7.55-7.50 (m, 1H), 7.48 (s, 1H), 7.45 (d, 2H, J = 4.4 Hz), 7.28-7.23 (m, 1H), 7.10-7.05 (m, 2H), 6.96-6.91 (m, 1H), 4.75 (d, 1H, J = 14.8 Hz), 4.46 (d, 1H, J = 14.8 Hz), 3.63 (d, 1H, J = 13.4 Hz), 3.52 (d, 1H J = 13.4 Hz), 3.50-3.45 (m, 2H), 3.08 (dd, 1H,J = 11.4, 3.5 Hz), 2.93-2.88 (m, 2H), 2.81 (d, 1H, *J* = 10.8 Hz), 2.76 (d, 1H, J = 11.0 Hz), 2.56 (dt, 1H, *J* = 11.9, 4.1 Hz), 2.40 (dt, 1H, J = 11.1, 2.3 Hz), 2.23 (dt, 1H, J = 10.9, 2.0 Hz), 2.15-2.10 (m, 1H).

Compound (15): 1H NMR (CDCl3, 500 MHz) : δ 7.21 (d, 2H, J = 8.2 Hz), 7.11 (d, 2H, J = 8.2 Hz), 4.58 (d, 1H, J = 14.7 Hz), 4.32 (d, 1H, J = 14.7 Hz), 3.51-3.44 (m, 2H), 3.07 (dd, 1H J = 4.1 & 11.5 Hz), 2.91-2.79 (m, 4H), 2.55 (dt, 1H, J = 4.3 & 11.9 Hz), 2.44-2.37 (m, 3H), 2.21 (t, 1H, J = 9.3 Hz), 2.17-2.04 (m, 2H), 2.01 (t, 1H, J = 10.8 Hz), 2.40 (dt, 1H, J = 11.1, 2.3 Hz), 2.23 (dt, 1H, J = 10.9, 2.0 Hz), 2.15-2.10 (m, 1H), 1.52-1.48 (m, 4H).

Compound (16): 1H NMR (CDCl3, 500 MHz) : δ 7.27-7.23 (m, 3H), 7.13 (t, 1H, J = 8.2 Hz), 4.67 (d, 1H, J = 14.8 Hz), 4.39 (d, 1H, J = 14.8 Hz), 3.51-3.44 (m, 2H), 3.07 (dd, 1H J = 4.1 & 11.5 Hz), 2.88 (dd, 1H, J = 4.1 & 11.5), 2.87-2.79 (m, 2H), 2.55 (dt, 1H, J = 4.3 & 11.9 Hz), 2.45-2.37 (m, 3H), 2.21 (t, 1H, J = 9.3 Hz), 2.17-2.04 (m, 2H), 2.01 (t, 1H, J = 10.8 Hz), 1.52-1.48 (m, 4H).

Compound (17): 1H NMR (CDCl3, 500 MHz) : δ 7.50 (d, 2H, J = 7.9 Hz), 7.28 (d, 2H, J = 7.9 Hz), 7.18 (q, 1H, J = 7.6 Hz), 7.00 (t, 2H, J = 6.9 Hz), 6.86 (t, 1H, J = 8.4 Hz), 4.64 (d, 1H, J = 14.8 Hz), 4.40 (d, 1H, J = 14.8 Hz), 3.56 (d, 1H, J = 13.4 Hz), 3.45 (d, 1H, J = 13.4 Hz), 3.43-3.39 (m, 2H), 3.00 (dd, 1H J = 3.8 & 11.5 Hz), 2.84-2.75 (m, 2H), 2.73 (d, 1H J = 10.8 Hz), 2.69 (d, 1H J = 11.0 Hz), 2.44 (dt, 1H, J = 4.3 & 11.9 Hz), 2.32 (t, 1H, *J =* 10.4 Hz), 2.13 (t, 1H, J = 8.8 Hz), 2.01 (t, 1H, J = 10.8 Hz).

Compound (18): 1H NMR (CDCl3, 500 MHz): δ 7.24-7.18 (m, 2H), 7.16-7.11 (m, 2H), 6.93-6.89 (m, 4H), 4.57 (d, 1H, J = 14.6 Hz), 4.28 (d, 1H, J = 14.6 Hz), 3.51 (d, 1H, J = 13.4 Hz), 3.41 (d, 1H, J = 13.4 Hz), 3.38-3.36 (m, 2H), 2.98 (dd, 1H J = 3.8 & 11.5 Hz), 2.82-2.77 (m, 2H), 2.71 (d, 1H, J = 10.9 Hz), 2.67 (d, 1H, J = 11.0 Hz), 2.44 (dt, 1H, J = 4.3 & 11.9 Hz), 2.28 (t, 1H, J = 10.4 Hz), 2.13 (t, 1H, J = 8.8 Hz), 2.01 (t, 1H, J = 10.8 Hz).

Compound (19): 1H NMR (CDCl3, 500 MHz): δ 7.50 (d, 2H, J = 7.9 Hz), 7.28 (d, 2H, J = 7.9 Hz), 7.21-7.16 (m, 3H), 7.13-7.08 (m, 2H), 4.62 (d, 1H, J = 14.9 Hz), 4.44 (d, 1H, J = 14.9 Hz), 3.45 (d, 1H, J = 11.1 Hz), 3.40 (dd, 1H, J = 3.8 & 11.5 Hz), 3.00 (dd, 1H J = 3.8 & 11.5 Hz), 2.87-2.78 (m, 2H), 2.75-2.71 (m, 2H), 2.56 (t, 2H, J = 7.6 Hz), 2.46 (dt, 1H, J = 4.3 & 11.9 Hz), 2.38 (t, 2H, J = 7.6 Hz), 2.39-2.33 (m, 1H), 2.23-2.15 (m, 1H), 1.96 (t, 1H, J = 8.8 Hz), 1.81-1.77 (m, 2H).

Compound (20): 1H NMR (CDCl3, 500 MHz): δ 7.33 (s, 1H), 7.28 (d, 2H, J = 8.0 Hz), 7.24-7.19 (m, 3H), 7.17 (d, 2H, J = 8.0 Hz), 4.66 (d, 1H, J = 14.9 Hz), 4.36 (d, 1H, J = 14.9 Hz), 3.60 (d, 1H, J = 11.1 Hz), 3.49 (d, 1H, J = 11.5 Hz), 3.47-3.41 (m, 2H), 3.04 (dd, 1H J = 3.8 & 11.5 Hz), 2.92-2.84 (m, 2H), 2.80 (d, 1H, J = 6.8 Hz), 2.74 (d, 1H, J = 6.8 Hz), 2.52 (dt, 1H, J = 4.3 & 11.9 Hz), 2.38 (t, 1H, J = 10.4 Hz), 2.22 (t, 1H, J = 8.8 Hz), 2.11 (t, 1H, J = 10.8 Hz).

Compound (21): 1H NMR (CDCl3, 500 MHz): δ 7.52 (s, 1H), 7.47-7.43 (m, 2H), 7.36 (t, 1H, J = 7.7 Hz), 7.15 (d, 1H, J = 5.5 Hz), 7.13 (d, 1H, J = 5.5 Hz), 6.92 (t, 2H, J = 8.7 Hz), 4.58 (d, 1H, J = 14.6 Hz), 4.30 (d, 1H, J = 14.6 Hz), 3.62 (d, 1H, J = 13.5 Hz), 3.51 (d, 1H, J = 13.5 Hz), 3.42-3.39 (m, 1H), 3.36 (dd, 1H, J = 5.1 & 11.8 Hz), 2.97 (dd, 1H, J = 4.1 & 11.6 Hz), 2.84-2.80 (m, 2H), 2.75-2.73 (m, 1H), 2.7.-2.67 (m, 1H), 2.46 (dt, 1H, J = 4.3 & 11.9 Hz), 2.33 (t, 1H, J = 10.4 Hz), 2.18 (t, 1H, J = 10.2 Hz), 2.08 (t, 1H, J = 11.0 Hz).

Compound (22): 1H NMR (CDCl3, 500 MHz): δ 7.25 (s, 1H), 7.19-7.10 (m, 5H), 6.92 (t, 2H, J = 8.5 Hz), 4.58 (d, 1H, J = 14.6 Hz), 4.30 (d, 1H, J = 14.6 Hz), 3.52 (d, 1H, J = 11.1 Hz), 3.40 (d, 1H, J = 11.1 Hz), 3.40-3.36 (m, 2H), 2.97 (dd, 1H J = 3.8 & 11.5 Hz), 2.81-2.80 (m, 2H), 2.72 (d, 1H, J = 6.8 Hz), 2.67 (d, 1H, J = 6.8 Hz), 2.45 (dt, 1H, J = 4.3 & 11.9 Hz), 2.30 (t, 1H, J = 10.4 Hz), 2.15 (t, 1H, J = 8.8 Hz), 2.03 (t, 1H, J = 10.8 Hz).

Compound (23): 1H NMR (CDCl3, 500 MHz): δ 7.50 (d, 2H, J = 7.9 Hz), 7.33 (S, 1H), 7.28 (d, 2H, J = 7.9 Hz), 7.24-7.20 (m, 3H), 4.62 (d, 1H, J = 14.9 Hz), 4.44 (d, 1H, J = 14.9 Hz), 3.60 (d, 1H, J = 11.1 Hz), 3.49 (d, 1H, J = 11.5 Hz), 3.47-3.41 (m, 2H), 3.04 (dd, 1H J = 3.8 & 11.5 Hz), 2.92-2.84 (m, 2H), 2.80 (d, 1H, J = 6.8 Hz), 2.74 (d, 1H, J = 6.8 Hz), 2.52 (dt, 1H, J = 4.3 & 11.9 Hz), 2.38 (t, 1H, J = 10.4 Hz), 2.22 (t, 1H, J = 8.8 Hz), 2.11 (t, 1H, J = 10.8 Hz).

Compound (24): 1H NMR (CDCl3, 500 MHz): δ 7.29 (d, 2H, J = 8.3 Hz),7.23-7.19 (m, 2H), 7.18 (d, 2H, J = 8.4 Hz), 7.19-7.16 (m, 3H), 4.58 (d, 1H, J = 14.6 Hz), 4.30 (d, 1H, J = 14.6 Hz), 3.53 (d, 1H, J = 11.1 Hz), 3.44 (dt, 1H, J = 4.9 & 11.8 Hz), 3.05 (dd, 1H J = 3.8 & 11.5 Hz), 2.89-2.80 m, 2H), 2.64 (t, 2H, J = 7.7 Hz), 2.52-2.50 (m, 1H), 2.41 (t, 2H, J = 7.7 Hz), 2.39 (t, 2H, J = 11.0 Hz), 2.19 (t, 2H, J = 10.7 Hz), 2.03 (t, 1H, J = 10.8 Hz), 1.89-1.84 (m, 2H).

Compounds (25): 1H NMR (CDCl3, 500 MHz): δ 7.30-7.25 (m, 2H), 7.23-7.19 (m, 2H), 7.19-7.16 (m, 3H), 7.00 (t, 2H, J = 8.6 Hz), 4.63 (d, 1H, J = 14.6 Hz), 4.41 (d, 1H, J = 14.6 Hz), 3.53 (d, 1H, J = 11.1 Hz), 3.44 (dt, 1H, J = 4.9 & 11.8 Hz), 3.05 (dd, 1H J = 3.8 & 11.5 Hz), 2.89-2.80 m, 2H), 2.64 (t, 2H, J = 7.7 Hz), 2.52-2.50 (m, 1H), 2.41 (t, 2H, J = 7.7 Hz), 2.39 (t, 2H, J = 11.0 Hz), 2.19 (t, 2H, J = 10.7 Hz), 2.03 (t, 1H, J = 10.8 Hz), 1.89-1.84 (m, 2H).

Compound (26): 1H NMR (CDCl3, 500 MHz): δ 7.32 (dd, 1H, J = 8.4 & 15.1 Hz), 7.23-7.16 (m, 3H), 6.88-6.83 (m, 1H), 6.84 (dt, 1H, J = 1.8 & 8.2 Hz), 6.80 (dt, 1H, J = 2.5 & 9.8 Hz), 4.60 (d, 1H, J = 14.7 Hz), 4.49 (d, 1H, J = 14.7 Hz), 3.55 (d, 1H, J = 13.4 Hz), 3.44 (d, 1H, J = 13.4 Hz), 3.42-3.35 (m, 2H), 2.98 (dd, 1H, J = 11.5, 3.9 Hz), 2.83-2.78 (m, 2H), 2.74-2.66 (m, 2H), 2.45 (dt, 1H, J = 12.0, 4.3 Hz), 2.31 (dt, 1H, J = 11.2, 2.7 Hz), 2.15 (dt, 1H, J =11.0, 2.5 Hz), 2.07-2.00 (m, 1H).

Compound (27): 1H NMR (CDCl3, 500 MHz): δ 7.68-7.61 (m, 1H), 7.67 (s, 1H), 7.56 (d, 1H, J = 7.2 Hz), 7.44 (t, 1H, J = 7.4 Hz), 7.32 (dd, 1H, J = 8.4 & 15.1 Hz), 6.84 (dt, 1H, J = 1.8 & 8.2 Hz), 6.80 (dt, 1H, J = 2.5 & 9.8 Hz), 4.60 (d, 1H, J = 14.7 Hz), 4.49 (d, 1H, J = 14.7 Hz), 3.73-3.67 (m, 2H), 3.54-3.49 (m, 2H), 3.16 (dd, 1H, J = 4.0 & 11.6 Hz), 3.15-2.87 (m, 2H), 2.91 (dd, 1H, J = 4.8 & 11.8 Hz), 2.84 (d, 1H, J = 11.0 Hz), 2.61-2.57 (m, 1H), 2.57-2.43 (m, 1H), 2.40-2.28 (m, 1H), 2.25-2.15 (m, 1H).

Compound (28): 1H NMR (CDCl3, 500 MHz): δ 7.86-7.82 (m, 1H), 7.67 (d, 1H, J = 7.6 Hz), 7.41 (d, 1H, J = 7.4 Hz), 7.36 (d, 1H, J = 13.7 Hz), 7.22 (d, 2H, J = 8.3 Hz), 7.10 (d, 2H, J = 8.3 Hz), 6.35 (brs, 1H), 5.64 (brs, 1H), 4.59 (d, 1H, J = 14.7 Hz), 4.30 (d, 1H, J = 14.7 Hz), 3.68 (d, 1H, J = 13.1 Hz), 3.57 (d, 1H, J = 13.1 Hz), 3.44-3.34 (m, 2H), 3.01-2.91 (m, 1H), 2.99 (dd, 1H, J = 4.0 & 11.6 Hz), 2.85-2.73 (m. 3H), 2.57-2.48 (m, 1H), 2.47-2.40 (m, 1H), 2.29-2.18 (m, 2H).

Compound (29): 1H NMR (CDCl3, 500 MHz): δ 7.25-7.23 (dd, 2H, J = 5.5 & 8.5 Hz), 7.03 (t, 2H, J = 8.7 Hz), 6.96-6.87 (m, 2H), 6.72 (t, 1H, J = 8.8 Hz), 4.68 (d, 1H, J = 14.5 Hz), 4.36 (d, 1H, J = 14.5 Hz), 3.72-3.62 (m, 1H), 3.61-3.42 (m, 3H), 3.08 (dd, 1H, J = 4.1 & 11.6 Hz), 3.00-2.85 (m, 1H), 2.90 (dd, 1H, J = 4.8 & 11.8 Hz), 2.85-2.78 (m, 2H), 2.57 (dt, 1H, J = 4.5 & 11.6 Hz), 2.49-2.39 (m, 1H), 2.37-2.25 (m, 1H), 2.21-2.13 (m, 1H).

Compound (30): 1H NMR (CDCl3, 500 MHz): δ 7.15 (d, 2H, J = 5.5 Hz), 7.13 (d, 2H, J = 5.5 Hz), 7.06 (s, 1H), 6.95-6.90 (m, 4H), 4.59 (d, 1H, J = 14.7 Hz), 4.29 (d, 1H, J = 14.7 Hz), 3.52 (d, 1H, J = 13.8 Hz), 3.44 (d, 1H, J = 13.8 Hz), 3.40-3.35 (m, 2H), 2.99 (dd, 1H, J = 4.0 & 11.6 Hz), 2.88-2.80 (m, 2H), 2.75-2.73 (m, 1H), 2.72-2.68 (m, 1H), 2.47 (dt, 1H, J = 4.1 & 11.6 Hz), 2.40-2.31 (m, 1H), 2.18 (t, 1H, J = 10.6 Hz), 2.07 (t, 1H, J = 10.5 Hz).

Compound (31): 1H NMR (CDCl3, 500 MHz): δ 7.21 (d, 2H, J = 8.4 Hz), 7.10 (d, 2H, J = 8.4 Hz),6.81 (d, 2H, J = 6.4 Hz), 6.63-6.60 (m, 1H), 4.59 (d, 1H, J = 14.7 Hz), 4.28 (d, 1H, J = 14.7 Hz), 3.56 (d, 1H, J = 12.4 Hz), 3.45 (d, 1H, J = 13.7 Hz), 3.41-3.37 (m, 2H), 3.00 (dd, 1H, J = 4.0 & 11.6 Hz), 2.83-2.80 (m, 2H), 2.74 (d, 1H, J = 10.9 Hz), 2.70-2.68 (m, 1H), 2.47 (dt, 1H, J = 4.3 & 11.6 Hz), 2.35-2.30 (m, 1H), 2.23-2.18 (m, 1H), 2.29-2.07 (m, 1H).

Compound (32): 1H NMR (CDCl3, 500 MHz): δ 7.51 (d, 2H, J = 9.0 Hz), 7.28 (d, 2H, J = 8.0 Hz), 6.81 (d, 2H, J = 6.4 Hz), 6.62 (d, 1H, J = 8.9 Hz), 4.66 (d, 1H, J = 14.6 Hz), 4.34 (d, 1H, J = 14.6 Hz), 3.53 (d, 1H, J = 10.9 Hz), 3.47-3.87 (m, 3H), 3.01 dd, 1H, J = 4.0 & 11.5 Hz), 2.85-2.81 (m, 2H), 2.75 (d, 1H, J = 10.9 Hz), 2.74-2.69 (m, 1H), 2.50 (dt, 1H, J = 4.3 & 11.9 Hz), 2.40-2.31 (m, 1H), 2.19 (t, 1H, J = 10.6 Hz), 2.07 (t, 1H, J = 10.6 Hz).

Compound (33): 1H NMR same as compound (1) as it is an enantiomer of (1)

Compound (34): 1H NMR same as compound (12) as it is an enantiomer of (12)

Compound (35): 1H NMR same as compound (12) as it is an enantiomer of (12)

Compound (36): 1H NMR same as compound (1) as it is an enantiomer of (1)

Compound (37): 1H NMR same as compound (29) as it is an enantiomer of (29)

Compound (38): 1H NMR same as compound (29) as it is an enantiomer of (29)

### Example 2.1.2: General method for the preparation of N2, N8 bis-substituted hexahydro-2H-pyrazino[1,2-a]pyrazin-1(6H)-one (d), wherein the two groups at N2 and N8 are identical.

To a stirring solution of (b1) in DMF (1 mL/0.1 mmol of b1) was added NaH (4.5 eq). After 15 minutes, a solution 4-(trifluoromethyl) benzyl bromide (2.2 eq) in DMF (0.5 mL) was added dropwise and the solution stirred for another hour. The solution was cooled to 0 °C, and water (2 ml/ 0.1 mmol) was added, and the solution was frozen. Solvent was removed by lyophilization overnight. The organics were dissolved in DCM and purified by column chromatography (DCM/MeOH 92:8). Removal of solvent afforded the desired product.

### 2.1.3 Preparation of 8-(4-trifluoromethylbenzyl)-2-(4-(trifluoromethyl)benzyl)hexahydro-2H-pyrazino[1,2-a]pyrazin-1(6H)-one (14).

Compound (14) was prepared using the general procedure described in part 2.1.2, using 4-(trifluoromethyl)benzyl bromide as the alkylating agent. Product was an off white solid. Yield = 52 %

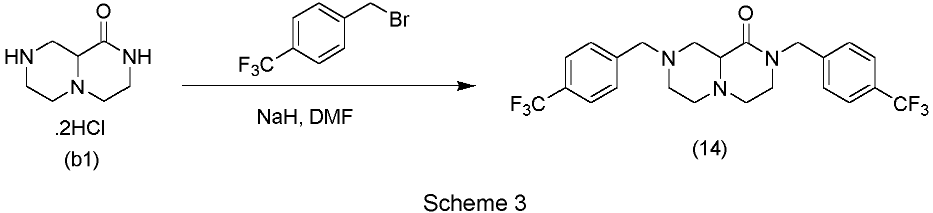

### EXAMPLE 3: Synthesis of HCl salts of compounds of formula (I).

### 3.1. General procedure for the preparation of hydrochloride salts of compounds of formula (I).

To compound of formula (I) was added a solution of HCl in methanol (1.25M, 5 eq) at RT. The solution was stirred for 1h, then the organics were removed under reduced pressure. If needed, diethyl ether was added to aid precipitation of the salt which was collected by filtration and dried in vacuo at 45 °C.

### 3.1.1. Preparation of 8-(3-fluorobenzyl)-2-(3-(trifluoromethyl)benzyl)hexahydro-2H-pyrazino[1,2-alpyrazin-1(6H)-one (13. 2HCl)

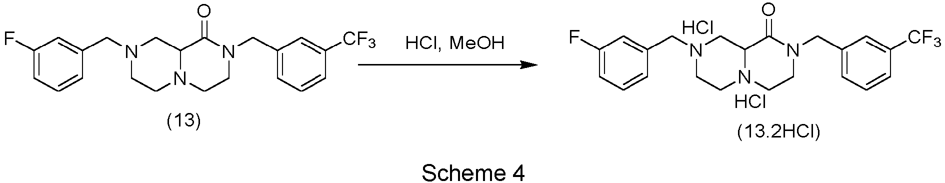

The titled compound was obtained using the general procedure described in example 3.1 Product (formula I. 2HCl) was obtained in quantitative yield as a white solid.

### EXAMPLE 4: Conversion of cyan substituted compounds of formula (I) into amido substituted compounds of formula (I).

### 4.1. General procedure for the conversion of cyan substituted compounds of formula (I) into amido substituted compounds of formula (I).

A mixture of cyano derivative (1.15 mmol) in acetic acid/sulphuric acid (1.4 mL; 1:1) was heated at 80 °C for 1h. After cooling to room temperature, the solution was poured onto ice (5g). The solution was basified to Ph = 9 with NaHCO₃, and the organics extracted with EtOAc (3 x 5 ml). The combined organics were washed with brine, dried and concentrated. The resulting solid was purified by column chromatography or crystallization, if neccessary.

### 4.1.1. Preparation of 3-((8-(4-chlorobenzyl)-9-oxooctahydro-2H-pyrazino[1,2-a]pyrazin-2-yl)methyl)benzamide (28).

Compound (28) was prepared using the general procedure described in part 3.1 [1 mmol of (3) was used], using 4-fluorobenzyl bromide as the initial alkylating agent and 3-cyanobenzyl bromide as the second alkylating agent. Product was a clourless white solid. Yield = 85 %

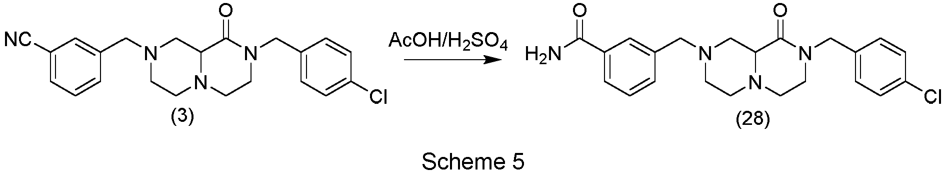

### EXAMPLE 5: Preparation of enantiomers of compounds of the formula (I)

### 5.1. General method for the preparation of enantiomers of compounds of the formula (I) using the synthetic method outlined in scheme B.

Sodium hydride (1.2 eq) was added to a solution of tert-butyl (S)-9-oxooctahydro-2H-pyrazino[1,2-a]pyrazine-2-carboxylate (1.0 eq) in DMF (3 mL/mmol) at O °C. After 15 mins, the appropriate alkylating reagent (Y-A-CH₂-Hal) (1.2 eq) was added and the mixture was allowed to warm to room temperature. After 2 h, TLC analysis indicated that the reaction was complete. The mixture was cooled to O °C and ice (5 g/mmol)) was added. The mixture was frozen and lyophilized to remove all solvents. The resulting solids were dissolved in DCM and filtered to remove salts. The organics were then concentrated to afford intermediate (g) as a colourless oil. The solids were dissolved in dioxane (3 mL/mmol) and 3 eq of HCl (solution in dioxane) were added. After 1 h the reaction was frozen and lyophilized to afford the Boc-deprocted intermediate, which was reacted with the desired alkylated agent (X-A-CH₂-Hal, 1.1 eq) in the presence of triethylamine (1.2 eq) in acetonitrile at RT for 3 h. The solvent was removed and replaced with EtOAc (10 mL/ mmol). The organics were washed with water (2 x 5 mL), then brine (10 mL). The organics were dried (Na₂SO₄) and concentrated. The residue was washed repeatedly with hexanes to remove any unreacted alkylating reagent.The desired products were generally colourless oils or white solids. If necessary, the products could be further purified by column chromatography using DCM/MeOH 95.5 as the solvent mixture.

### 5.1.1. Preparation of (S)-3-((8-(4-fluorobenzyl)-9-oxooctahydro-2H-pyrazino[1,2-a]pyrazin-2-yl)methyl)benzonitrile (36)

Compound (36) was prepared using the general procedure described in part 4.1 [1 mmol of (f) was used], using 4-fluorobenzyl bromide as the initial alkylating agent and 3-cyanobenzyl bromide as the second alkylating agent. Product was a colourless white solid. Yield = 33 %

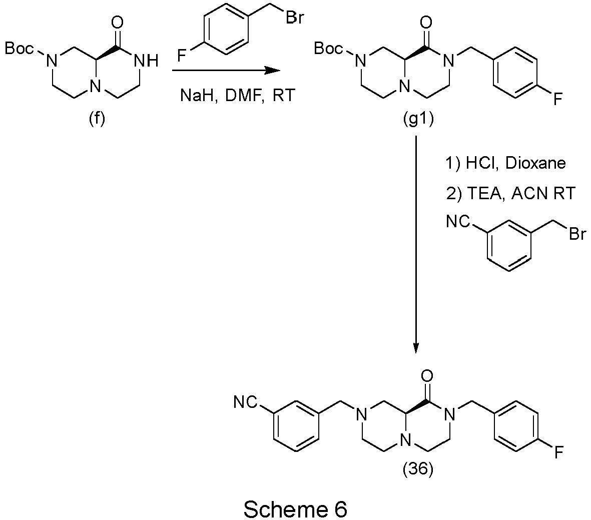

### EXAMPLE 6: Biological experiments

Experiments were performed as shown below for the compounds of formula (I) of present invention prepared in the examples described above herein.

### 6.1. Method used to identify selective human ClpP activators using genetically modified yeast.

### 6.1.1. Preparation of yeast, with or without the presence of human ClpP, that have several efflux pumps down regulated.

### Strain construction

Yeast cells expressing hClpP were constructed by using the *Modular Cloning* (MoClo) method (10). This technique allows to directionally assemble several DNA fragments into one single DNA piece. It is based on plasmid libraries that share the same DNA backbone but differ in the type of the insert. The assembly of the final cassette is based on the *"Golden Gate Cloning"* method. Human *CLPP* gene sequence (codon-optimized for yeast translation) was amplified with well-designed primers from a plasmid kindly provided by Dr. Michael David Tyers (University of Montreal). Once amplified, a MoClo reaction was performed to obtain the final integrative cassette. This cassette was composed as follows:
- Type 1: *ConLS* 3' connector.
- Type 2: *TDH3* gene promoter (strong and constitutive expression).
- Type 3: Amplified human *CLPP* gene.
- Type 4: *ADH1* gene terminator.
- Type 5: ConR1 5' connector.
- Type 6: *LEU2* gene as selectable marker for yeast.
- Type 7: 3' HO gene homology sequence for yeast integration.
- Type 8a: *KanR-ColE1* sequence for bacterial amplification and antibiotic resistance.
- Type 8b: 5' HO gene homology sequence for yeast integration.

After plasmid assembly reaction and subsequent transformation in *DH5α Escherichia coli,* large amount of the plasmid was obtained by *Maxiprep.* Digestion patterns with several restriction enzymes were carried out to confirm the success of the MoClo system. An extra restriction enzyme reaction (*Notl*) was performed to linearize the DNA fragment and deplete the bacterial part. Linearization gave rise to a lineal fragment flanked by 5' and 3' homology sequences to the HO gene, which further served for chromosomic integration at the mutated HO locus.

The used yeast strain contains the following background:
*MATa trp1-1 leu2-3,112 his3-11,15 ura3-1 ade2-1 can1-100 yrs1::HIS3 yrr1::TRP1 pdr1::hisG pdr3::hisG.*

These cells contain mutants in *YRS1, YRR1, PDR1* and *PDR3* genes, which are involved in polydrug and pleiotropic resistance networks. These mutants significantly reduce the capability of the yeast to remove xenobiotic compounds.

Yeast cells were transformed with the linearized cassette and transformant colonies were selected on minimum media without leucine. Independent clones were checked by PCR and positive ones were stored at -80 °C until use.

### 6.1.2. Screening of compounds to identify activators of human ClpP.

The yeast (both HsCplP+ and HsClpP-) are incubated in petri dishes with YPD (Yeast extract peptone dextrose) at 25 °C overnight, after which a colony is isolated, suspended in a flask with fresh YPD and incubating again under the same conditions but with agitation. The culture was then diluted with YPD until an absorbance reading of 0.1 was achieved at 600 nM. 100 µl of this solution was then placed into the appropriated wells of a standard 96-well plate. Stock solutions (DMSO, 10 mM) of the compounds to be tested are diluted in YPD to 2X of their final concentrations to be tested, and 100 µl added to the appropriate wells. 100 µl of YPD was added to wells designated as the negative control wells, while ONC-206 (0.5 µM) was used as the positive control. Once all compounds were added to the plate, an initial T = 0 absorbance value was recorded at 600 Nm. The plate was then incubated at 25 °C with gentle shaking for 24 h. The plates can either be read at multiple time points using a TECAN Spark^{®} Multimode Microplate Reader, or at the end of the experiment using a standard spectrophotometer. The 50% growth inhibition concentration (Gl50) was calculated using the GraphPad 5.0 program.

Table 2 below sets forth potencies of representative compounds of the invention to inhibit yeast growth via modulating HsClpP.None of the compounds represented in table 2 inhibited the growth of yeast not possessing HsClpP at concentrations up to 10000 nM.

**Table 2: Growth inhibition (Gl50) values for compounds of the present invention in yeast containing HsClpP, and yeast without HsClpP.**

| ID# (table 1) | Yeast + HsClpP GI50 (nM) | Yeast - HsClpP GI50 (nM) | ID# (table 1) | Yeast + HsClpP GI50 (nM) | Yeast - HsClpP GI50 (nM) |
|---|---|---|---|---|---|
| ONC-201 | 10000 | >10000 | 19 | 592 | >10000 |
| ONC-206 | 4560 | >10000 | 20 | 67 | >10000 |
| 1 | 150 | >10000 | 21 | 71 | >10000 |
| 2 | 120 | >10000 | 22 | 35 | >10000 |
| 3 | 65 | >10000 | 23 | 5 | >10000 |
| 4 | 210 | >10000 | 24 | 206 | >10000 |
| 5 | 140 | >10000 | 25 | 381 | >10000 |
| 6 | 60 | >10000 | 26 | 608 | >10000 |
| 7 | 2050 | >10000 | 27 | 677 | >10000 |
| 8 | 1650 | >10000 | | | |
| 9 | 690 | >10000 | | | |
| 10 | 180 | >10000 | | | |
| 11 | 620 | >10000 | | | |
| 12 | 75 | >10000 | | | |
| 13 | 40 | >10000 | | | |
| 14 | 2580 | >10000 | | | |
| 15 | 721 | >10000 | | | |
| 16 | 1850 | >10000 | | | |
| 17 | 65 | >10000 | | | |
| 18 | 798 | >10000 | | | |

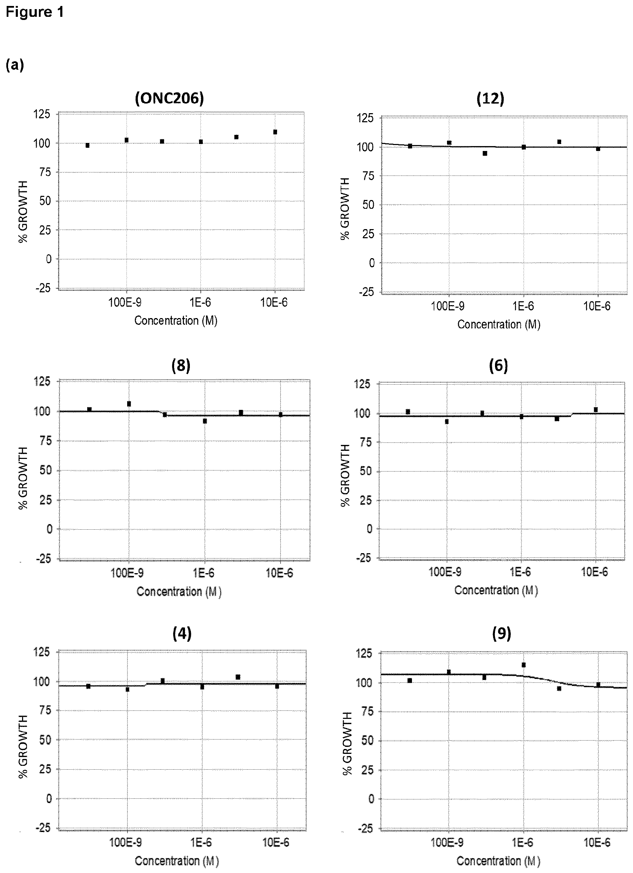

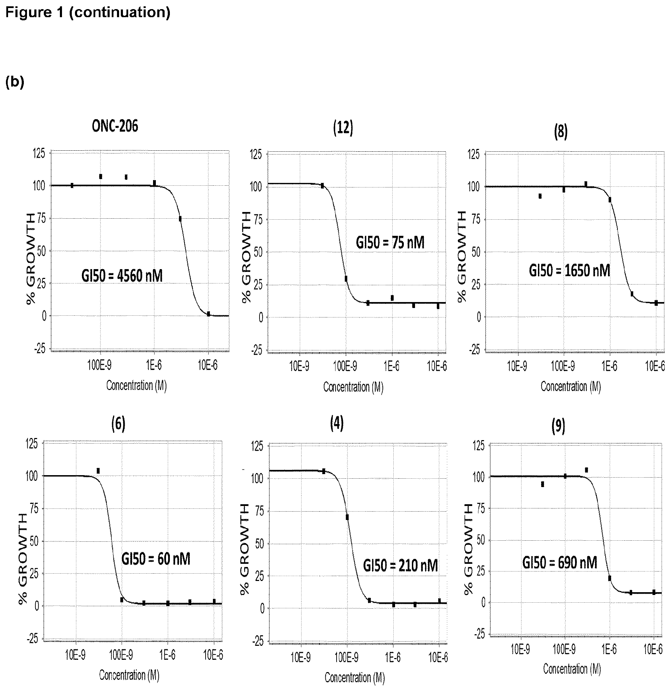

Moreover, Figure 1 shows, how compounds of the invention selectively inhibit the growth of yeast that contain HsClpP and are not toxic to yeast without HsClpP present. Furthermore, several compounds of the invention are more potent in this assay compared to both of the ClpP agonists in human clinical trials, ONC-201 and ONC-206

### 6.1.3. The effects of the compounds of the present invention on the cell viability of various human and murine derived cancer cell lines.

### Cell lines.

DU-145 [American Type Culture Collection (ATCC) reference number HTB-81]. This a human prostate cancer cell line derived from a metastatic site (brain) of a 68-year-old male.

PANC-1 [American Type Culture Collection (ATCC) reference number CRL-1469]. This a human cancer cell line derived from the pancreas of a 56-year-old male.

MDA-MB-231 [American Type Culture Collection (ATCC) reference number HTB-26]. This a human breast cancer cell line derived from a metastatic site from a 51-year-old female.

4T1 [American Type Culture Collection (ATCC) reference number CRL-2539]. This a mouse epithelial breast cancer cell line classified as being triple negative and mimics human breast cancer; stage IV.

U2 OS [American Type Culture Collection (ATCC) reference number HTB-96]. This is a human bone cancer (a moderately differentiated sarcoma) cell derived from a female osteosarcoma patient.

DLD-1 [American Type Culture Collection (ATCC) reference number CCL-221]. This is a colorectal adenocarcinoma cell line isolated from the large intestine of a colon adenocarcinoma patient.

### MTT Cell viability assay.

Cell viability was assayed by measuring the mitochondrial reduction of the tetrazolium salt 3-(4,5-methyltiazol-2yl)-2,5diphenyl-tetrazolium bromide] (MTT) (Applichen; A2231,0010) see (11) and (12). Cells were seeded in 96-well plates in medium (either RPMI, DMEM or McCoys depending on the recommendations from the ATCC) supplemented with 10% FBS (Lonza Cat. N°: 14-502F), 2mM L-Glut (Lonza Cat. N°: BE17-605E) and 100U/ml PEST (Lonza Cat. N°: DE17-605E) at a cell density of 1000-5000 cells/well (96 well plate). Cells were seeded at a density according to their exponential growth phase (normally 1000 to 5000/well). The cells were cultured at 37 °C in the dark in air containing 5% CO₂. After 24h either vehicle (0.05% DMSO), or drug, was added to culture medium at the indicated concentrations (10 to 0,01microM). After another 72 h, MTT (0.3 mg/ml) was added to cells, followed by incubation for 2-4h at 37 °C in the dark. The medium was then removed, and the precipitated formazan was solubilized by the addition of DMSO (200 µL).

The optical density was measured at 595 nm with the iMark Microplate Reader (BioRad, USA). Data was expressed as percent growth above the level in controls and the results in figures were plotted as means ± SEM of each test point from 3 replicates.

Statistical analysis. The concentration of drug that provokes 50% reduction of cell viability (IC₅₀) was determined by using non-linear regression analysis and data were fitted to a log concentration vs normalized response curve in GraphPad software v5 (San Diego, CA).

Table 3 below sets forth potencies of representative compounds of the invention in a variety of human or mouse derived cancer cell lines (values are averages of at least two independent experiments).

**Table 3: Compounds of the invention (formula I) reduce the cell viability in a collection of human or mouse cancer cell lines.**

| ID# (Table 1) | IC₅₀ (nM) 4T1 48 H | IC₅₀ (nM) MDA-MB-231 72 H | IC₅₀ (nM) U2 OS 72 H | IC₅₀ (nM) DU-145 72 H | IC₅₀ (nM) PANC1 72 H | IC₅₀ (nM) DLD-1 72 H |
|---|---|---|---|---|---|---|
| ONC-201 | 2215 | 2285 | 4800 | 1710 | 1720 | |
| ONC-206 | 600 | 600 | 800 | 370 | 300 | |
| 1 | 103 | 120 | 220 | 200 | 195 | <100 |
| 2 | 165 | 145 | 250 | 250 | 140 | - |
| 3 | <100 | <100 | <100 | <100 | <100 | < 100 |
| 4 | 330 | 450 | 500 | 850 | 335 | 438 |
| 5 | 190 | 240 | 400 | 300 | 250 | 168 |
| 6 | 150 | 190 | 300 | 250 | 190 | 161 |
| 7 | 340 | 430 | 500 | 500 | 330 | 419 |
| 8 | 310 | 400 | 2000 | - | 350 | 341 |
| 11 | 515 | 490 | 800 | 410 | 600 | 582 |
| 12 | <100 | <100 | 200 | 100 | <100 | < 100 |
| 13 | 170 | 190 | 300 | 250 | 165 | 123 |
| 15 | 171 | 364 | - | - | - | 206 |
| 16 | 451 | 487 | - | - | - | 607 |
| 17 | 343 | 409 | - | - | - | 515 |
| 18 | 507 | 490 | - | - | - | - |
| 19 | 574 | 448 | - | - | - | 500 |
| 20 | < 100 | < 100 | - | - | - | < 100 |
| 21 | < 100 | < 100 | - | - | - | < 100 |
| 22 | < 100 | < 100 | - | - | - | < 100 |
| 23 | < 100 | 158 | - | - | - | 207 |
| 24 | < 100 | 167 | - | - | - | 132 |
| 25 | < 100 | < 100 | - | - | - | < 100 |
| 29 | < 100 | < 100 | - | - | - | <100 |
| 30 | - | < 100 | - | - | - | < 100 |
| 31 | - | 164 | - | - | - | < 100 |
| 32 | - | 215 | - | - | - | < 100 |
| 33 | - | 157 | - | - | - | 195 |
| 34 | - | 164 | - | - | - | 100 |
| 35 | - | < 100 | - | - | - | < 100 |
| 36 | - | < 100 | - | - | - | < 100 |
| 37 | - | < 100 | - | - | - | < 100 |
| 38 | - | 210 | - | - | - | 310 |
| | | | | | | |
| | | | | | | |
| | | | | | | |

| | | | | | | |
|---|---|---|---|---|---|---|
| - represents compound not tested. | | | | | | |

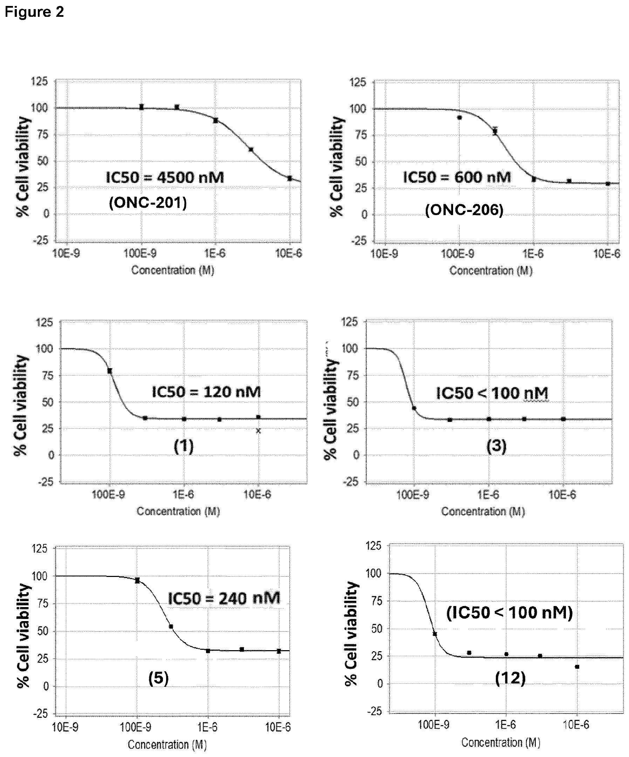

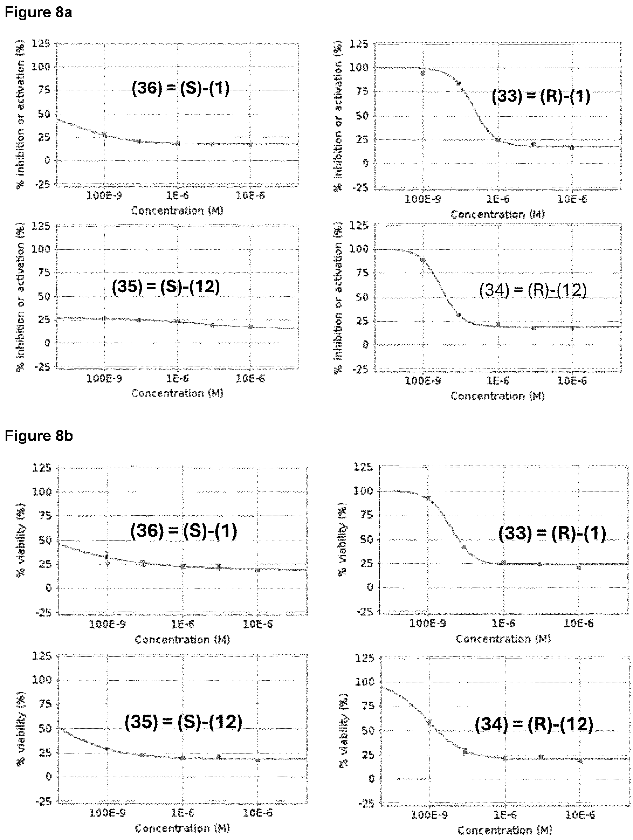

Moreover, Figure 2 shows that several compounds of the invention are more potent against MDA-MB-231 cells, than the two ClpP agonists currently in human clinical trials, ONC-201 and ONC-206. Furthermore, Figure 8a shows that the S-enantiomers of compound (1) [(36)] and compound (12) [(35)] had lower IC50 values (more potent) than their respective R-enantiomers (33) and (34). Also Figure 8b shows that the S-enantiomers of compound (1) [(36)] and compound (12) [(35)] had lower IC50 values (more potent) than their respective R-enantiomers (33) and (34).

Therefore, Figure 8 shows that, consistently with the activation data presented in table 6, although both enantiomers of compounds (1) [R = (33), S = (36)] and (12) [R = (34) and S = (35)] are potent inhibitors of cell growth of the colon cancer cell line DLD-1, the S-enantiomers are even more potent than their respective R-enantiomers.

According to the clinical trial website www.clinicaltrial.gov accessed on March 9th 2023, ONC-201 is in 9 active or recruiting clinical trials related to cancer, and ONC-206 is in 2 active or recruiting clinical trials related to cancer.

### 6.1.4. The effects of the compounds of the present invention on the cell growth of various human and murine derived cancer cell lines.

### SRB cell growth assay.

Sulforhodamine B (SRB) binds selectively to cellular proteins and can therefore be used to measure the effects of compounds on cellular growth. The method described here (modified from Houghton, P., et. al., Methods 42 (2007) 377-387) uses sulforhodamine B (SRB) as a means to analyze the effects of compounds on adherent cells using a 96-well format.

In order to measure the cell population at the time zero (Tz) a plate of cells was stained with SRB 24 hours after seeding. The cell monolayers are fixed with cold 5% (w/v) trichloroacetic acid for 60 minutes at 4°C. The excess of trichloroacetic acid is removed by washing five times with distilled water. Sulforhodamine B (SRB) solution [50 µL at 0.4%(w/v) in 1% acetic acid] is added to each well and plates are incubated for 8 minutes at room temperature. The excess dye is removed by washing five times with 1% (v/v) acetic acid. The bound stain is subsequently solubilized with (100 µL) 10 mM trizma base and the absorbance is read on automated plate reader at a wavelength of 550 nm.

Compounds at various concentrations were added to an identical cell-seeded (non-treated) plate and incubated at 37°C (5% CO2) for an additional 48 hours. Cells were fixed and stained following the same protocol. The measurement of the cell population after 72 hours in the presence of the drug is (Ti). Several wells in the same plate were treated with the control vehicle. The resulting measurements give the control growth value (C) after 72 h.

Using the absorbance measurements, time zero (Tz), control growth (C), and test growth in the present of the compound (Ti) the percentage growth inhibition is calculated as: $\left[\left(Ti-Tz\right)/\left(C-Tz\right)\right] \times 100 for concentrations for which Ti > / = Tz$ $\left[\left(Ti-Tz\right)/Tz\right] \times 100 for concentrations for which Ti < Tz$

### Statistical analysis.

Based on these values for each test concentration, the following parameters can be calculated:
- Growth inhibition concentration (Gl): concentration of compound required to reduce growth by 50%
- Total growth inhibition concentration (TGI): concentration of compound required to reduce growth by 100%
- Lethal concentration (LC): concentration of compound required to kill 50% of the initial cell population.

These values were determined using GraphPad software v5 (San Diego, CA).

**Table 4: Compounds of the invention (formula I) reduce the ability of MDA-MB-231 cells to grow.**

| | MDA-MB-231 72 H | | | |
|---|---|---|---|---|
| ID# (Table 1) | GI₅₀ (nM) | TGI₅₀ (nM) | LC₅₀ (nM) | Result |
| ONC-201 | 2500 | >10000 | >10000 | Cytostatic |
| ONC-206 | 730 | >10000 | >10000 | Cytostatic |
| 3 | <100 | >10000 | >10000 | Cytostatic |
| 12 | <100 | >10000 | >10000 | Cytostatic |
| 13 | 420 | >10000 | >10000 | Cytostatic |

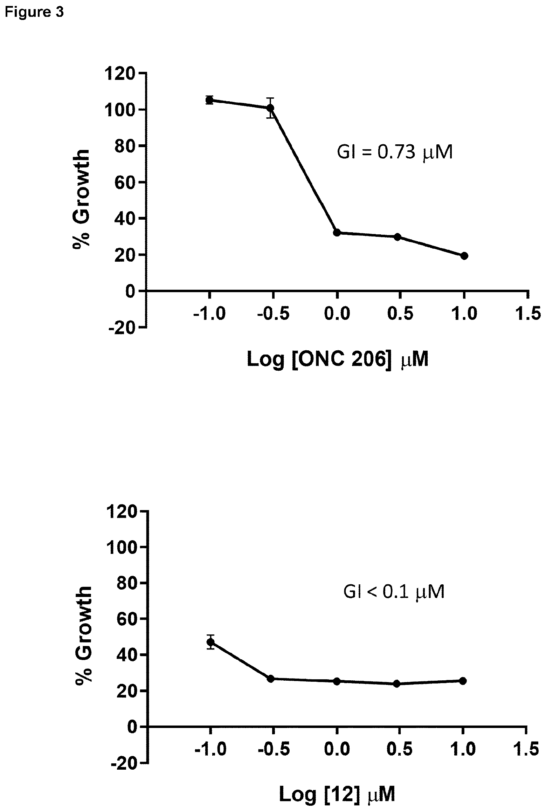

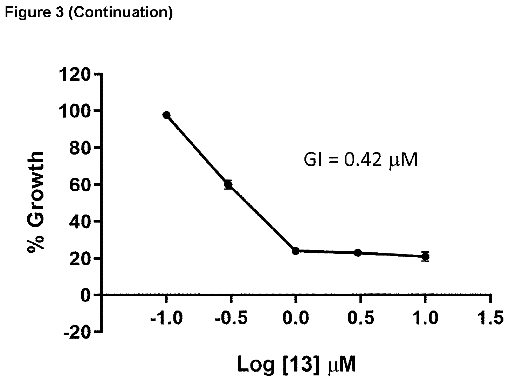

Additionally, Figure 3 also shows that compounds (12) and (13) of the present invention reduce the growth of MBA-MB-231 cells, and are cytostatic agents.

### 6.1.5. Effect of compounds of the present invention on tumours induced in immunocompetent mice using the murine TNBC cell line 4T1.

4T1 syngeneic tumours were induced in female immunocompetent Balb/c mice by inject 12500 4T1 cells (passage# <7, in PBS, 100 µL) into their left inguinal mammary fat pad. Once tumour volumes [measure using the formula V = 0.5(length x width²)] reached 100 m³, treatment with either vehicle, or compound in vehicle, was began. Compounds were administered orally (PO) at a constant dose (typically 25 mg/kg) three times per week (Monday, Wednesday and Friday). Compounds (as ether their free base or salt) were formulated as either solutions or homogenous suspensions in a vehicle of 10% Cremaphor RH40 + 90% saline for injection. Formulations were prepared at concentrations (mg/mL) = Dose/5 (e.g. a dose of 25 mg/Kg used a formulation of 5 mg/ml). Once treatments began, tumour volumes and body weights were recorded on each treatment day. Treatments were continued until 25-28 days after first treatment day, after which the mice were sacrificed. Animals whose tumour reached >1500 m³ during the experiment were also sacrificed.

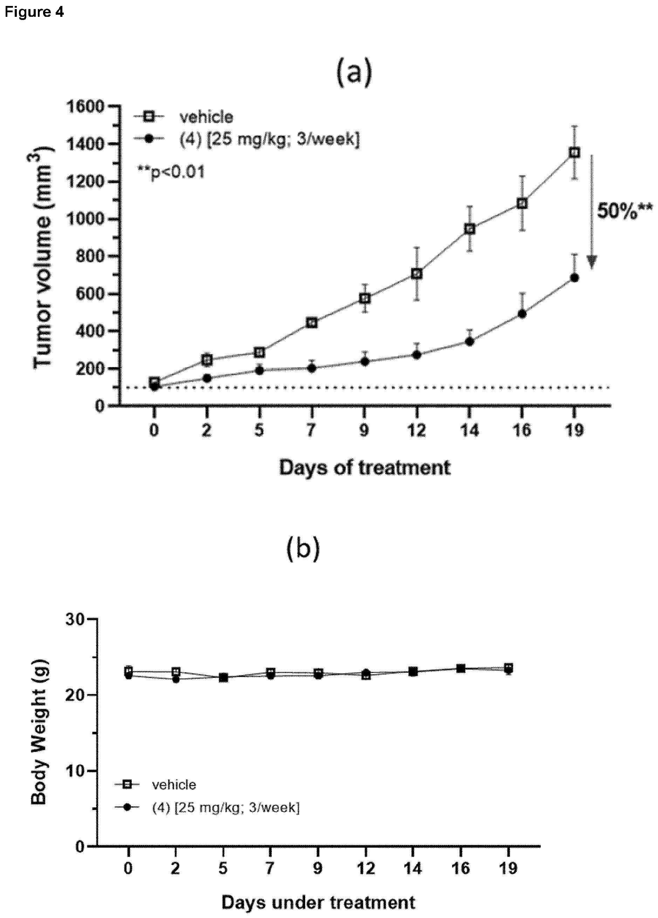

Figure 4a shows the effects of treating mice bearing established 4T1 tumours with either vehicle (10% creamphor in saline), or with 25 mg/kg of compound (4), three time per week. Tumour growth was significantly reduced (50%, p < 0.01) in those mice treated with compound (4), compared to animals treated with just vehicle. Also, treatment with compound (4) caused no significant changes in body weights during the experimental period (Figure 4b).

### 6.2. Method used to identify activators of human ClpP (HsClpP) and S.aureus ClpP (SaClpP) using recombinant proteins.

Without the presence of an activator, HsClpP and SaClpP can only degrade FITC-casein at a relatively slow rate. FITC-casein has a relatively low baseline level of fluorescence due to internal quenching of the fluorescence signal. However, upon degradation into smaller fragments this quenching effect is removed, and fluorescence increases proportional to the amount of degradation. The amount of activation is therefore relative to the intensity of the fluorescence. 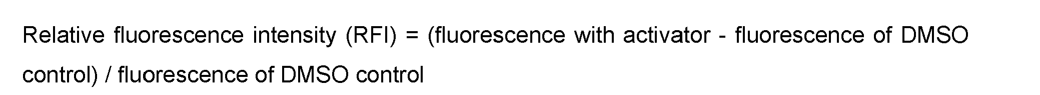 Relative fluorescence intensity (RFI) = (fluorescence with activator - fluorescence of DMSO control) / fluorescence of DMSO control

Compounds with RFI values ≥ 1.3 are deemed to be activators of that specific ClpP protein.

### 6.2.1. Protein degradation of FITC-casein by the activation of either HsClpP or SaClpP protein by compounds of the present invention.

This assay was based on the method published by Wei, B., et al. (7). To a buffer solution (containing 25 mM HEPES, pH 7.5, 200 mM KCI, 5 mM MgCl2, 10% (v:v) glycerol, 2 mM DTT) in a 384 well plate, was added the ClpP protein to be tested, to afford a final monomeric concentration of 2.5 µM. Compounds to be tested were then added to afford final concentrations between 10 and 0.1 micromolar. After that, FITC-casein was added to afford a final concentration 10 µM. The plate was then warmed at 37 °C for 70min. The fluorescence was measured at T = 0 and T = 70 min. DMSO was used as the negative control.

**Table 5: RFI values for compounds of the present invention, and the known activators of HsClpP (ONC-201 and ONC-206) after incubation with HsClpP or SaClpP and FITC-casein for 70 min, using various concentrations of the compounds of the present invention.**

| ID# (table 1) | HsClpP RFI at 10 µM | HsClpP RFI at 1 µM | SaClpP RFI at 10 µM | SaClpP RFI at 1 µM |
|---|---|---|---|---|
| ONC-201 | 3.6 | 2.6 | 2.2 | 1.0 |
| ONC-206 | 3.3 | 2.7 | 2.8 | 1.4 |
| 12 | 3.5 | 3.0 | 1.2 | 1.0 |
| 14 | 3.8 | 2.3 | 1.3 | 0.9 |
| 29 | 4.9 | 3.7 | 3.3 | 1.6 |
| 30 | 3.5 | 2.9 | 2.8 | 1.4 |
| 31 | 3.5 | 2.8 | 2.8 | 1.2 |
| 32 | 3.3 | 2.8 | 2.8 | 1.2 |
| 33 | 2.6 | 1.6 | 1.0 | 1.0 |
| 34 | 2.8 | 1.4 | 1.1 | 1.0 |
| 35 | 3.3 | 2.4 | 1.0 | 1.0 |
| 36 | 3.2 | 3.5 | 1.1 | 1.0 |
| 37 | - | - | 3.3 | 1.6 |
| 38 | - | - | 3.0 | 1.4 |

| | | | | |
|---|---|---|---|---|
| - represents not tested. | | | | |

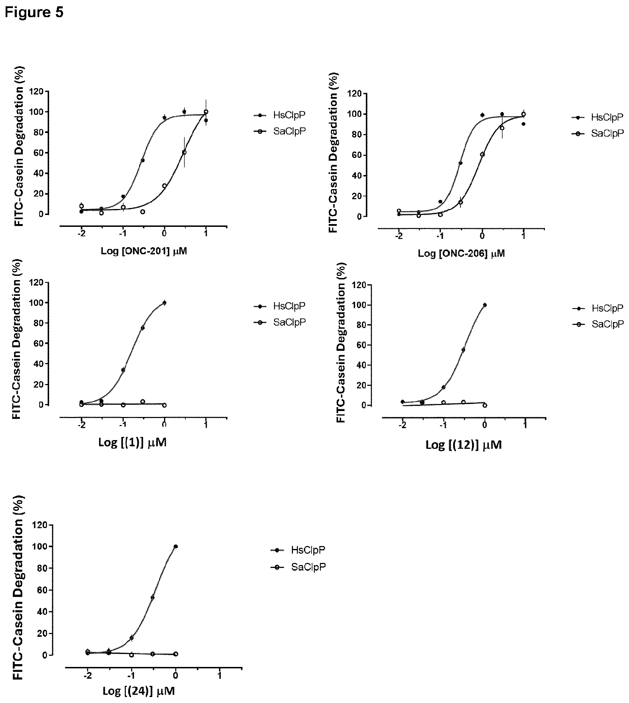

Moreover, Figure 5 shows that while ONC-201 and ONC-206 activate both HsClpP and SaClpP, many of the compounds of the invention only activate HsClpP. While most compounds of the invention preferentially activate HsClpP over SaClpP, those compounds possessing a 3,5-substitution pattern on the benzyl amine appendage differ in that they also significantly activate SaClpP. Compounds (29, 30, 31, 32, 37 and 38) are examples of compounds that can activate SaClpP to promote the degradation of FITC-casein. Furthermore, this data clearly shows that the (S)-enantiomers, compounds (35) and (36) are stronger activators of HsClpP than their (R)-enantiomeric versions, compounds (34) and (33) respectively.

### 6.3. Direct interaction and stabilization of SaClpP or HsClpP protein by compounds of the present invention.

Compounds that interact with a given protein in a stabilizing manner will generate a [compound-protein] combination that is more stable to heat than the protein alone. The more stable is the [compound-protein] complex the more affinity the compound has to bind to the protein. The thermal shift assay utilizes this theory to determine the binding capabilities of individual compounds. Compounds that increase the Tm (Melt temperature) the most, relative to the Tm of the protein alone, have a high binding affinity. Hence compounds with larger ΔTm values have higher binding affinities. $ΔTm = Tm \left(compound-protein\right) - Tm \left(DMSO-protein\right)$

A ΔTm > 5 °C is deemed to represent a meaningful stabilization of the protein.

### 6.3.1. Thermal shift assay to assess binding of compounds of the present invention to HsClpP or SaClpP.

To a buffer solution (containing 10 mM HEPES pH 7; 150mM NaCl, Protein Thermal Shift Dye 5X and Protein Thermal Shift Buffer 5uL for 20 uL assay) in 96 well plate, was added SaClpP or HsClpP to afford a final monomeric concentration of 10 µM. Compounds to be tested were added to afford final concentration 100 micromolar. The plate was warmed at 25 °C for 30 mins. After that, the samples were heated using a temperature ramp (2 °C / min) from 25 to 95 °C. The fluorescence was measured every 2 min. DMSO was used as a negative control. The raw fluorescence/temperature data was converted to a d(fluorescence)/dT v T plots using Protein Thermal Shift software from which the desired Tm could be extracted.

**Table 6: The change in melt temperature produced by the addition of selected compounds of the invention to either HsClpP, or SaClpP protein.**

| ID# (table 1) | HsClpP ΔTm (°C) | SaClpP ΔTm (°C) |
|---|---|---|
| ONC-201 | 13.7 | 8.1 |
| ONC-206 | 16.5 | 14.3 |
| 1 | 17.4 | 2.1 |
| 12 | 17.3 | 1.9 |
| 29 | 19.8 | 10.5 |
| 30 | 18.7 | 8.0 |
| 31 | 19.1 | 11.1 |
| 32 | 16.2 | 9.8 |

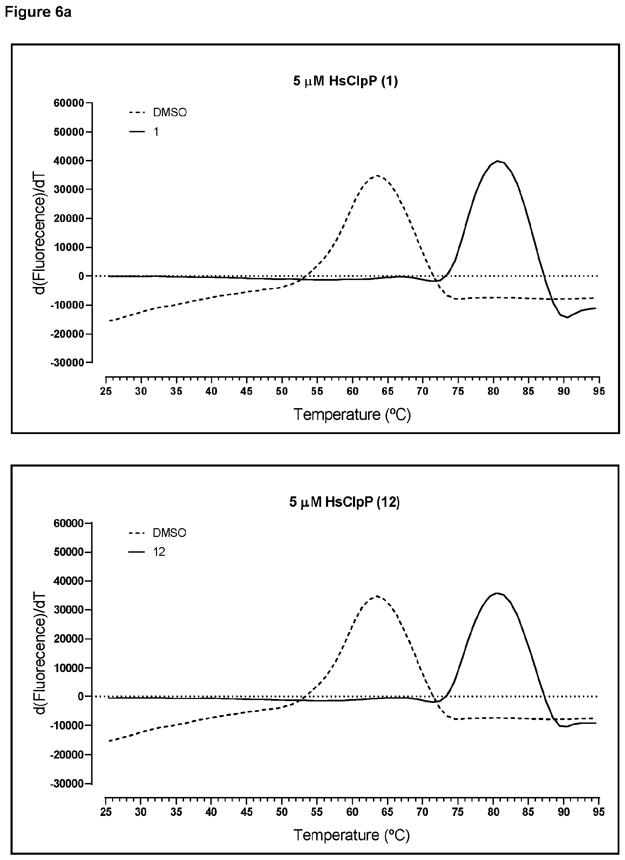

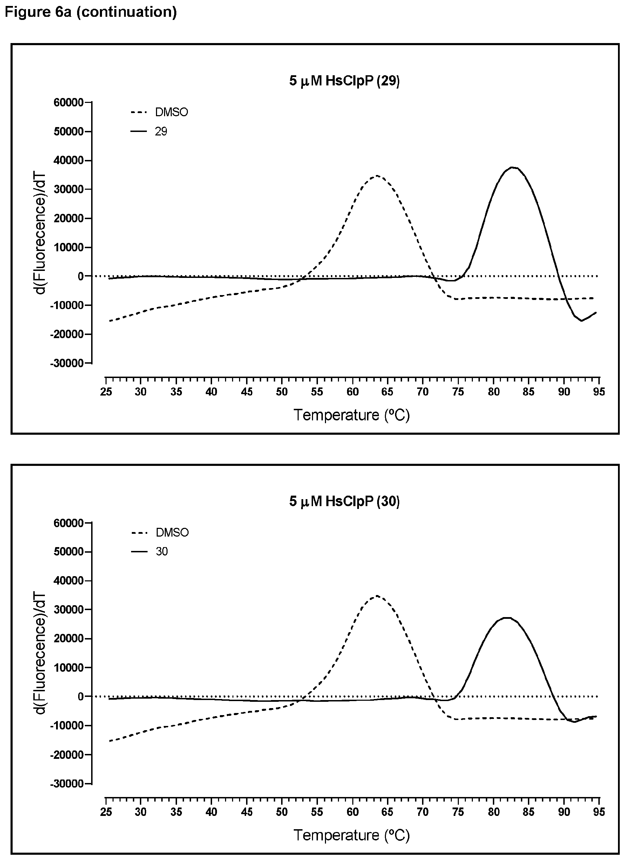

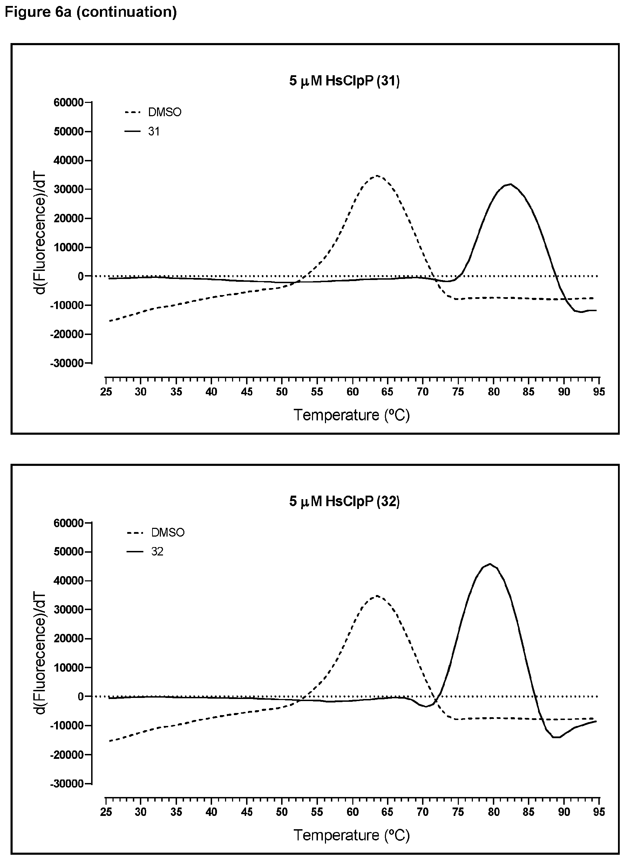

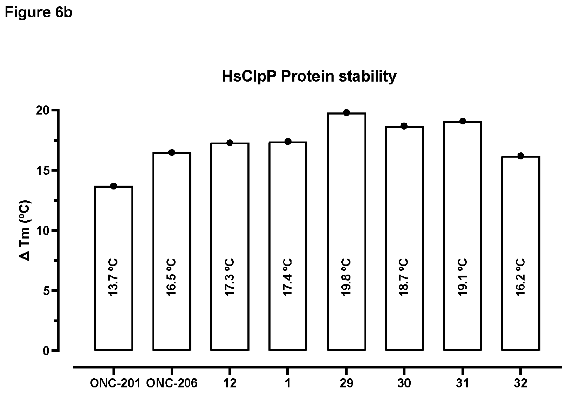

Figure 6 shows that Compounds (1), (12), (29), (30) and (31) all generated higher ΔTm values than both ONC-201 and ONC-206, while compound (32) had a ΔTm value higher than ONC-201 and comparable to that of ONC-206.

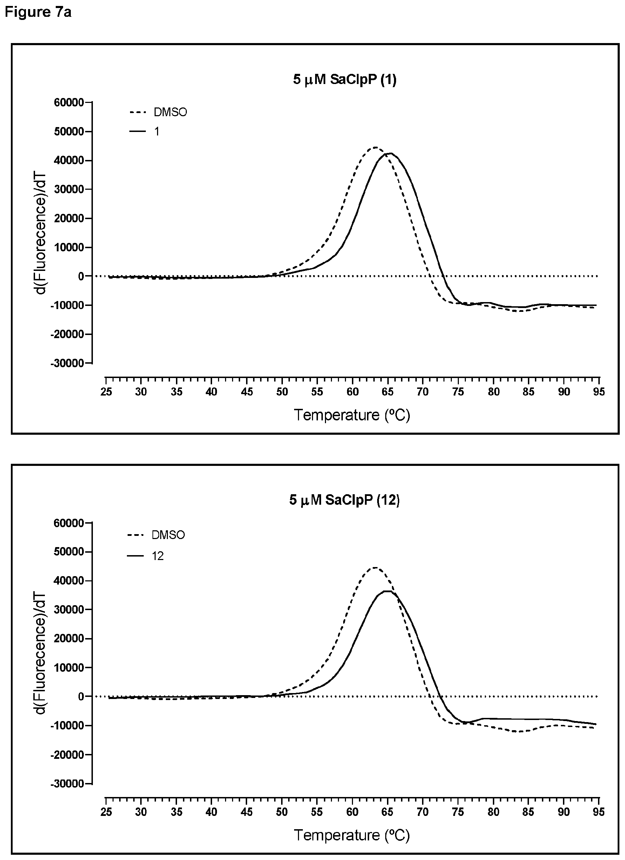

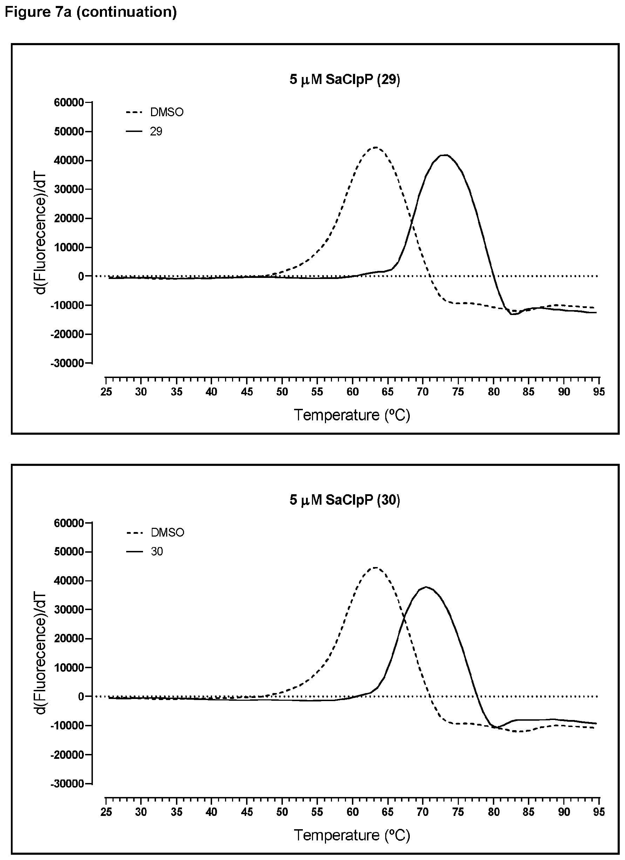

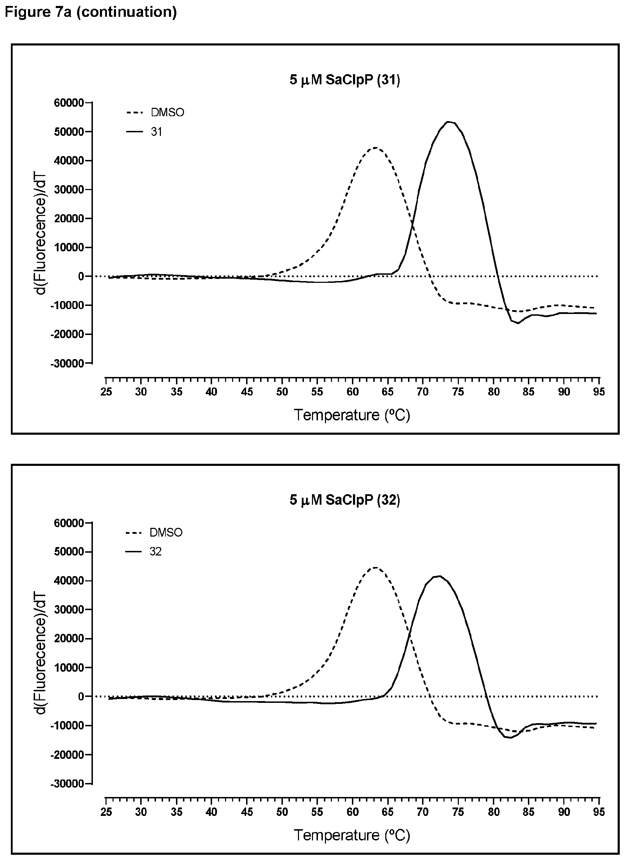

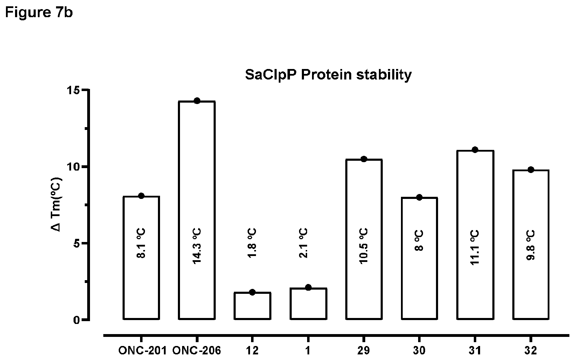

Figure 7 shows that as predicted from the activation assay (SaClpP-FITC assay) compounds (1) and (12) have low ΔTm values and therefore do not significantly stabilize the SaClpP protein, while compounds (29), (30), (31) and (32) demonstrated large ΔTm values, indicating that they could stabilize the SaClpP protein. ΔTm values for compound (30) was comparable to that observed for ONC-201 while the ΔTm values for compounds (29), (31) and (32) were greater than that generated by ONC-201.

In agreement with the previous ClpP activation data (table 6) compounds that only activated the HsClpP protein, such as compounds (1) and (12), only generated a meaningful thermal shift with HsClpP, and not with SaClpP (Figures 6 and 7). While compounds that activated both HsClpP and SaClpP, such as (29, 30, 31 and 32) and the two ONC compounds, were capable of stabilizing both forms ClpP tested (Figures 6 and 7). Of note, not only were compounds (1) and (12) more selective for HsClpP than the two ONC compounds but they also generated larger ΔTm values.

### 6.4. Antibacterial effects of compounds of the present invention.

6.4.1. Antibacterial effects of compounds of the present invention on a methicillin-sensitive strain of *Staphylococcus aureus* (MSSA) bacteria (ATCC25923).

First, the bacteria are cooled in a petri dish with TSA (Tryptic soy agar) and incubated for 24h at 37 °C to obtain isolated colonies. After this time, a colony is selected and inoculated into a flask with TSB (Tryptic soy broth) medium, incubating again under the same conditions but with agitation overnight. The resulting culture was diluted until an absorbance reading at 600nm was equal to 0.01. 180 µl of this culture solution was added to the designated positions within a standard 96-well plate. Stock solutions (DMSO, 10 mM) of the compounds to be tested were diluted in TSB to 10X of their final concentrations to be tested, and 20 µl was added to the appropriate wells. 20 µl of TBS was added to the bacterial culture as a negative control, while Vancomycin was used as a positive (4 µg/ml) control for *S.aureus.*

The absorbance was of each well was recorded at 600 nM to obtain their T = 0 value. The plate was shaken gently at 37 °C for 18 h, after which the absorbance was recorded (T = 18 h). Compounds of the invention were first tested at a single concentration (60 µM). Compounds that reduced bacterial growth by ≥ 50% were then tested from 60 - 3.75 µM using serial dilution methods. An IC50 value for each compound was then determined.

Table 7 sets forth the % of growth reduction observed using S. aureus (ATCC25923) bacteria after being treated with compounds of the present invention at 60 µM. Compounds are capable of reducing the bacterial growth by ≥ 50% at 60 µM, had their IC50 determined.

**Table 7: Selected compounds of the invention can reduce the growth of S.aureus bacteria.**

| ID# | % reduction of growth at 60 µM | IC50 (µM) |
|---|---|---|
| 4 | 6 | - |
| 5 | 25 | - |
| 6 | 52 | 60 |
| 8 | 14 | - |
| 10 | 22 | - |
| 13 | 57 | 30 |
| 15 | 7 | - |
| 16 | 8 | - |
| 17 | 50 | 60 |
| 18 | 5 | - |
| 20 | 17 | - |
| 21 | 10 | - |
| 22 | 16 | - |
| 23 | 12 | - |
| 24 | 5 | - |
| 26 | 10 | - |
| 27 | 7 | - |
| 28 | 4 | - |
| 29 | 67 | 18 |
| 30 | 64 | 24 |
| 31 | 79 | 9 |
| 32 | 82 | 10 |
| 37 | 71 | 30 |
| 38 | 31 | - |

| | | |
|---|---|---|
| - represents compound not tested. | | |

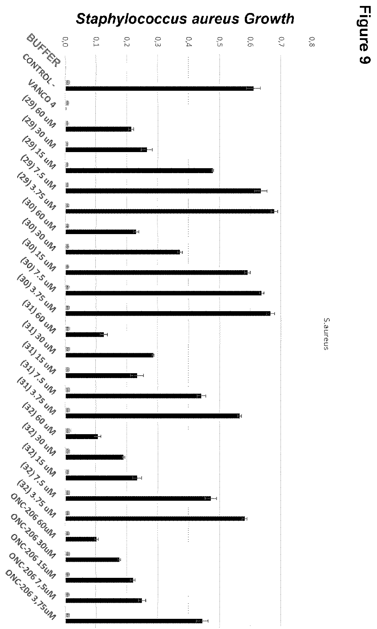

Compounds capable of stabilizing and activating SaClpP were shown to be able to reduce the growth of S.aureus bacteria. Moreover, Figure 9, demonstrates the concentration-dependent effects of compounds (29), (30), (31), (32) and ONC-206 on the inhibition of growth of S.aureus bacteria, and therefore that compounds of the invention are able to reduce the growth of S. aureus bacteria in a concentration-dependent manner.

### 6.5. Effect of compound (4) on tumours induced in immunocompetent mice using the murine TNBC cell line 4T1.

4T1 syngeneic tumours were induced in female immunocompetent Balb/c mice by inject 12500 4T1 cells (in PBS, 100 µL) into their left inguinal mammary fat pad. Once tumour volumes [measure using the formula V = 0.5(length x width²)] reached 100 m³, treatment with either vehicle, or compound in vehicle, was began. Compounds were administered orally (PO) at a constant dose (typically 25 mg/kg) three times per week (Monday, Wednesday and Friday). Compounds (as ether their free base or salt) were formulated as either solutions or homogenous suspensions in a vehicle of 10% Cremaphor RH40 + 90% saline for injection. Formulations were prepared at concentrations (mg/mL) = Dose/5 (e.g. a dose of 25 mg/Kg used a formulation of 5 mg/ml). Once treatments began, tumour volumes and body weights were recorded on each treatment day. Treatments were continued until 25-28 days after first treatment day, after which the mice were sacrificed. Animals whose tumour reached >1500 m³ during the experiment were also sacrificed.

Figure 4a shows the effects of treating mice bearing established 4T1 tumours with either vehicle (10% creamphor in saline), or with 25 mg/kg of compound (4), three time per week. Tumour growth was significantly reduced (50%, p < 0.01) in those mice treated with compound (4), compared to animals treated with just vehicle. Also, treatment with compound (4) caused no significant changes in body weights during the experimental period (Figure 4b).

### 6.6. Stability of compounds of the invention to human liver microsomes.

Compounds that are more stable to the metabolic actions of microsomes are more likely to survive hepatic degradation and enter into circulation in the blood stream and be available for distribution to the desired target.

### 6.6.1. Determination of the intrinsic clearance and half-life of compounds of the invention in the presence of human liver microsomes.

A solution of the test compound (final concentration of 0.1 micromolar) is added to human liver microsomes (0.1 mg/mL). At time intervals 0, 15, 30, 45 and 60 mins after addition of the test compound, aliquots of the mixture are extracted and analyzed by LC-MS-MS to determine the quantity of test compound present. Metabolic stability, expressed as percent of the parent compound remaining, was calculated by comparing the peak area of the compound at the time point relative to that at time = 0. The half-life (T1/2) was estimated from the slope of the initial linear range of the logarithmic curve of compound remaining (%) vs. time, assuming the first-order kinetics. The apparent intrinsic clearance (CLint, µL/min/mg) was calculated according to the following formula: $Clint = 0.693 / {T}_{1 / 2} * \left(mg protein/μL\right)$

**Table 8: Intrinsic clearance and half-life data for compounds of the invention when treated with human liver microsomes.**

| ID | Clint (µL/min/mg) | Half-life (mins) |
|---|---|---|
| ONC-201 | 745 | 9 |
| 1 | 75 | 93 |
| 2 | 300 | 23 |
| 3 | 226 | 31 |
| 4 | 407 | 17 |
| 5 | 158 | 44 |
| 6 | 301 | 23 |
| 7 | <58 | >120 |
| 8 | 95 | 73 |
| 9 | 214 | 32 |
| 10 | 301 | 23 |
| 11 | < 58 | >120 |
| 12 | 154 | 45 |
| 14 | 492 | 14 |
| 17 | 395 | 17 |
| 18 | 118 | 59 |
| 20 | 423 | 16 |
| 21 | 590 | 12 |
| 24 | 330 | 21 |
| 27 | 71 | 99 |

Several compounds of the invention are significantly more stable to metabolic degradation by human liver microsomes than the currently most advanced ClpP activator in clinical trials, ONC-201.

### 6.7. Cardiac safety assessment in vitro.

The inhibition of cardiac ion channels can detrimentally affect the generation and maintenance of cardiac action potentials which are essential for a heart to perform correctly. Of the several ion channels involved in the initiation and maintenance of the action potential the potassium channel hERG is known to play an important role in the repolarization of the action potential. Many natural and synthetic compounds are known to inhibit hERG and cause cardiac toxicity. Therefore, the ability of selected compounds of the invention to inhibit the hERG ion channel was assessed using patch clamp electrophysiology and cells over-expressing hERG channels.

### 6.7.1. Inhibition of the cardiac potassium ion channel hERG using patch-clamp electrophysiology.

After whole cell configuration is achieved, the cell is held at -80mV. The cell is depolarized to +40mV for 500ms and then to -80mV over a 100ms ramp to elicit the hERG tail current. This paradigm is delivered once every 8s to monitor the current amplitude. The parameters measured were the maximum tail current evoked on stepping to 40mV and ramping back to - 80mV from the test pulse. All data were filtered for seal quality, seal drop, and current amplitude. The peak current amplitude was calculated before and after compound addition and the amount of block was assessed by dividing the Test compound current amplitude by the Control current amplitude. Control data is the mean hERG current amplitude collected 15 seconds at the end of the control period; Test compound data is the mean hERG current amplitude collected 15 seconds at the end of test concentration application for each concentration.

All compounds were tested in the presence of 0.1% Pluronic F-68 Non-Ionic Surfactant and at room temperature.

**Table 9: % Inhibition of hERG current after treating with 10 micromolar test compound or the calculated IC50 (micromolar) from using at least three different concentrations.**

| ID | % Inhibition of hERG current | IC50 (µM) |
|---|---|---|
| 1 | 26.4 ± 1.0 | >10 |
| 2 | 40.0 ± 0.2 | >10 |
| 3 | 46.0 ± 0.2 | 10.3 |
| 4 | 44.3 ± 4.1 | >10 |
| 5 | 35.5 ± 0.5 | >10 |
| 6 | 54.6 ± 0.1 | 9.8 |
| 9 | 46.9 ± 1.5 | 10.3 |
| 10 | 67.4 ± 0.2 | - |
| 11 | 28.1 ± 0.3 | >10 |
| 12 | 48.1 ± 0.6 | 9.9 |
| 17 | 50.7 ± 1.7 | 9.8 |
| 18 | 36.6 ± 1.0 | >10 |
| 20 | 34.9 ± 0.7 | >10 |
| 21 | 38.9 ± 1.1 | >10 |

| | | |
|---|---|---|
| - represents compound not tested. | | |

Data from table 9 highlights that several compounds of the invention possess much lower inhibitory effects on the potassium cardiac ion channel hERG, suggesting a lower probability of cardiac toxicity.

### 6.8 Comparison of compounds with amino core (Y=H,H) versus compounds with amido core (Y= O).

In order to determine whether compounds with amido core would be active against HsClpP and in cancer, compounds 1 and 12 were tested with the amido and amino core.

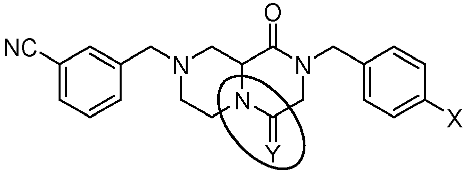

### 6.8.1. Increase in activation of HsClpP.

**Table 10. Increase in activation of HsClpP by compounds 1 and 12 and their respective analogues with amido core. Values ≥ 1.5 signify an increase in activation.**

| ID | X | Y | 3 mM | 1 mM | 0.3 mM | 0.1 mM |
|---|---|---|---|---|---|---|
| 12 | CF3 | H,H | 4.0 | 3.0 | 2.1 | 1.3 |
| 12' | CF3 | O | 2.9 | 1.4 | 1.1 | 1.0 |
| 1 | F | H,H | 3.8 | 3.0 | 1.8 | 1.1 |
| 1' | F | O | 2.8 | 1.2 | 1.2 | 1.1 |

Data from table 10 shows that compounds of present invention activate HsClpP at 10 times lower concentrations than analogue compounds with amido core.

### 6.8.2 Cell growth inhibition.

**Table 11. Cell growth inhibition by compounds 1 and 12 and their respective analogues with amido core.**

| ID | X | Y | DLD-1 GI50 (nM) | MDA-MB-231 GI50 (nM) | SKOV-3 GI50 (nM) |
|---|---|---|---|---|---|
| 12 | CF3 | H,H | 77 | <100 | 214 |
| 12' | CF3 | O | 7570 | 5430 | 5720 |
| 1 | F | H,H | 65 | 237 | <100 |
| 1' | F | O | 5220 | 4320 | ND |

Data from table 11 shows that amino core is **>18 times** more active than the amido core in MDA-MB-231 cells, **>25 times** in SKOV-3 cells and **>80 times** more active in DLD-1 cells.

Furthermore, data also showed that compounds with the amido core are **cytotoxic,** while compounds of the invention are **cytostatic.**

### References

1. Mabanglo, M.F., et al., Substrates and interactors of ClpP protease in the mitochondria, Curr. Opin. Chem. Biol., 2022, 66:102078; doi: 10.1016/j.cbpa.2021.07.003.
2. Wang, P., et al., Aberrant human CIpP activation disturbs Mitochondrial proteome homeostasis to suppress pancreatic ductal adenocarcinoma, Cell Chem. Biol., 2022, 29, 1396-1408; doi: 10.1016/j.chembiol.2022.07.002.
3. Luo, B., et al., Human CIpP protease, a promising therapy for diseases of mitochondrial dysfunction., Drug Dis.Today., 2021 26(4), 968-981; doi: 10.1016/j.drudis.2021.01.007.
4. Wong, K. S., et al., Acyldepsipeptide Analogs Dysregulate Human Mitochondrial HsClpP Protease Activity and Cause Apoptotic Cell Death. Cell Chem. Biol. 2018, 25, 1017-1030.e9; doi: 10.1016/j.chembiol.2018.05.014.
5. Prabhu, V.V., et al., ONC201 and impridones: Anti-cancer compounds with clinical efficacy, Neoplasia, 2020, 22(12), 725-744; doi: 10.1016/j.neo.2020.09.005.
6. Fatima, N., et al., The CIpP activator, TR-57, is highly effective as a single agent and in combination with venetoclax against CLL cells in vitro, Leuk Lymphoma. 2024, 65(5), 585-597; doi: 10.1080/10428194.2023.2300055.
7. Wei, B., et al., Anti-infective therapy using species-specific activators of Staphylococcus aureus ClpP, Nat. Comm., 2022, 13, 6909; doi.org/10.1038/s41467-022-34753-0.
8. Dunn, R.L., et al., Eds. POLYMERIC DRUGS AND DRUG DELIVERY SYSTEMS, ACS Symposium Series Vol. 469, American Chemical Society, Washington, D.C. 1991.
9. Greene, T.W., et al., "Protective Groups in Organic Synthesis", 3rd Ed., Wiley & Sons, Inc, New York (1999); doi: 10.1002/0471220574.
10. Lee, M.E., et al., Highly Characterized Yeast Toolkit for Modular, Multipart Assembly. ACS Synth Biol. 2015, 4(9), 975-86; doi: 10.1021/sb500366v.
11. Mosmann T. Rapid colorimetric assay for cellular growth and survival: application to proliferation and cytotoxicity assays. J Immunol Methods. 1983, 65(1-2), 55-63; doi: 10.1016/0022-1759(83)90303-4.
12. Chuan, Y. C., et al., Ezrin mediates c-Myc actions in prostate cancer cell invasion. Oncogene. 2010, 29(10), 1531-42; doi: 10.1038/onc.2009.442.

## Claims

1. A compound of formula (I), or a pharmaceutically acceptable salt or solvate thereof: 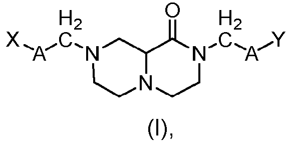 wherein;
A is independently selected from the group consisting of: C₁-C₆ alkyl or is absent;
X and Y are independently selected from the group consisting of C₁-C₆ haloalkyl, aromatic carbocyclic 6-membered ring, or an aromatic heterocyclic 5- or 6-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
R⁷ substituent is independently selected from the group consisting of: H, halogen, C₁-C₆ haloalkyl, -O-C₁-C₆ alkyl, -O-C₁-C₃ haloalkyl, cyano, C₁-C₆ alkyl and -C(O)OR⁸;
R⁸ is independently selected from the group consisting of: C₁-C₆ alkyl, C₃-C₆ cycloalkyl or C₁-C₆ aminoalkyl.

2. A compound of formula (I), or a pharmaceutically acceptable salt or solvate thereof, according to claim 1, wherein:
A is independently selected from the group consisting of: C₁-C₆ alkyl or is absent;
X and Y are independently selected from the group consisting of C₁-C₆ haloalkyl, aromatic carbocyclic 6-membered ring, or an aromatic heterocyclic 5- or 6-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
wherein R⁷ substituent is independently selected from the group consisting of: H, halogen, C₁-C₆ haloalkyl, -O-C₁-C₆ alkyl, -O-C₁-C₂ haloalkyl, cyano and C₁-C₆ alkyl.

3. A compound of formula (I), or a pharmaceutically acceptable salt or solvate thereof, according to claims 1 or 2, wherein:
A is independently selected from the group consisting of: C₁-C₃ alkyl or is absent;
X and Y are independently selected from the group consisting of C₁-C₃ haloalkyl, aromatic carbocyclic 6-membered ring, or an aromatic heterocyclic 5-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
wherein R⁷ substituent is independently selected from the group consisting of: H, F, Cl, Br, C₁-C₃ haloalkyl, -O-C₁-C₆ alkyl, -O-CF₃, cyano and C₁-C₆ alkyl.

4. A compound according to claim 1 or 2, having formula (II), or a pharmaceutically acceptable salt or solvate thereof: 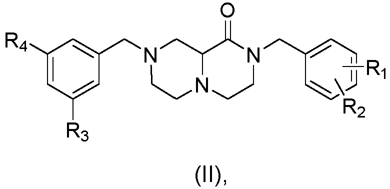 wherein;
R¹ and R² substituents are independently selected from the group consisting of: H, halogen and CF₃;
R³ and R⁴ substituents are independently selected from halogen or CF₃.

5. A compound or pharmaceutical acceptable salt or solvate thereof according to any one of claims 1 to 4, wherein the compound is selected from:

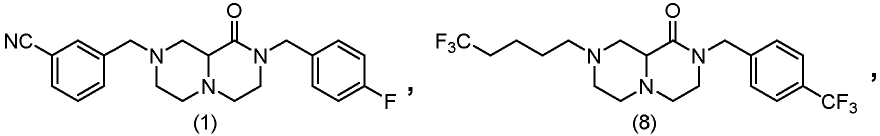

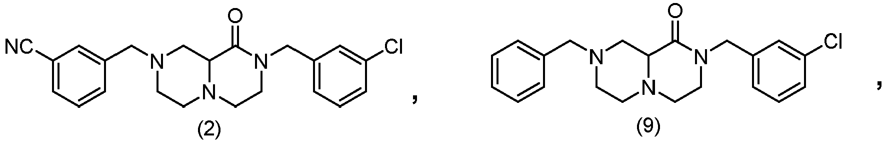

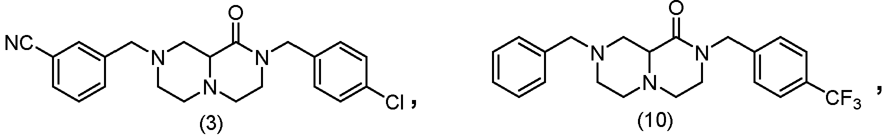

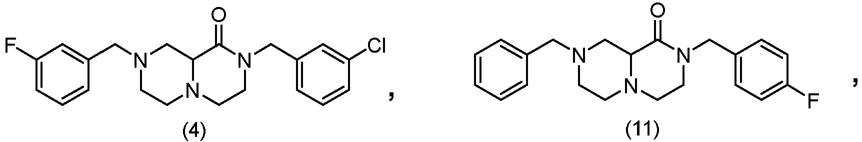

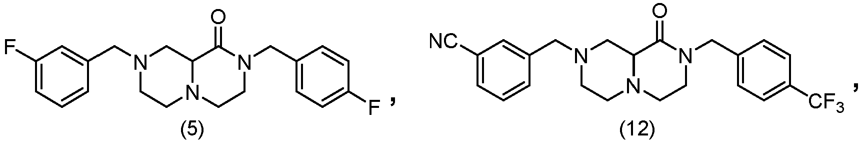

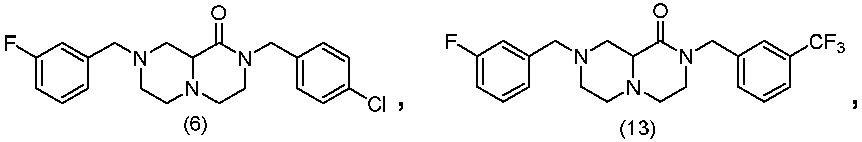

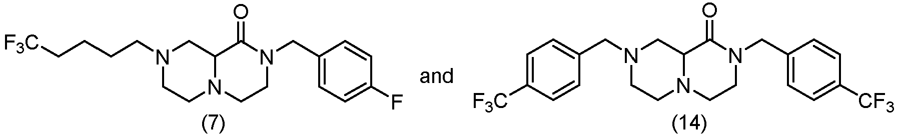

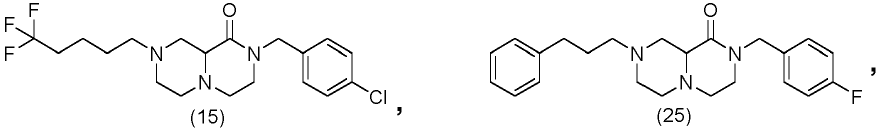

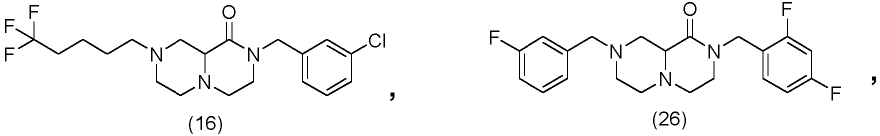

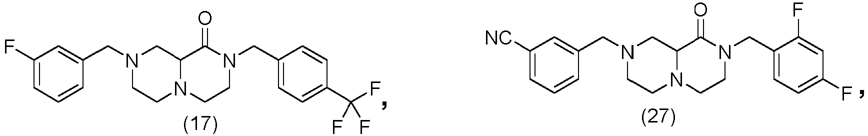

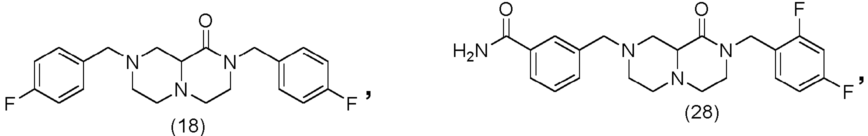

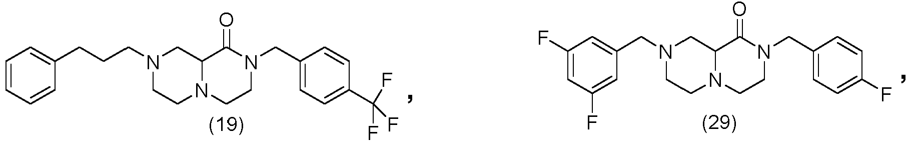

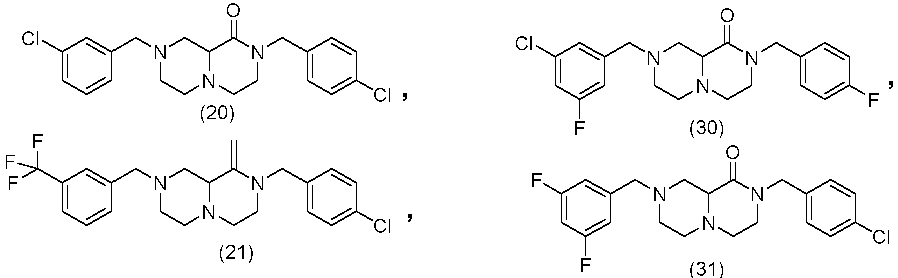

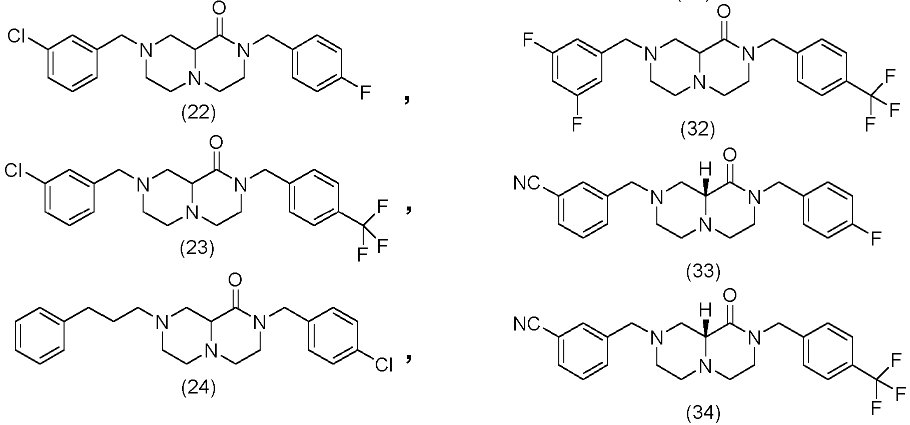

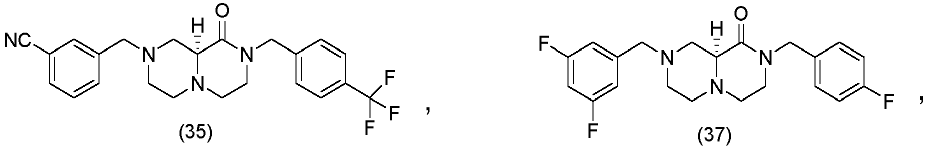

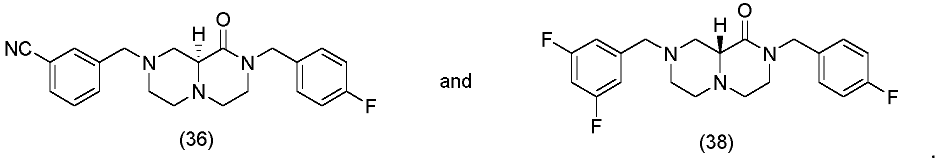

6. A compound of formula (I) or formula (II), or pharmaceutical acceptable salt or solvate thereof, according to any one of claims 1 to 5, wherein the compound, the salt, or the solvate comprise stereoisomers, polymorphs, isotopes, or in the form of complexes with antibodies.

7. A pharmaceutical composition comprising at least one compound of formula (I) or formula (II), according to any one of claims 1 to 6, and at least one pharmaceutically acceptable excipient or carrier.

8. A pharmaceutical composition according to claim 7, further comprising at least one additional active compound or agent selected from the group consisting of a chemotherapeutic, a cell therapy, an immunotherapeutic or an antibiotic.

9. A compound of formula (I) or formula (II), according to any one of claims 1 to 6, or a pharmaceutical composition according to claim 7 or 8, for use as a medicament.

10. A compound of formula (I) or formula (II), according to any one of claims 1 to 6, or a pharmaceutical composition according to any of claims 7 or 8 for use in the prevention and/or treatment of cancer.

11. A compound or pharmaceutical composition for use according to claim 10, wherein the cancer is selected from the group consisting of: sarcoma, breast cancer, prostate cancer, head and neck cancer, brain tumour, colorectal cancer, lung cancer, pancreatic cancer, cervical cancer, ovarian cancer, gastric cancer, renal cell carcinoma, melanoma, endometrial cancer, hepatocellular carcinoma, chronic myelogenous leukaemia, acute myelogenous leukaemia, cutaneous T-cell lymphoma, Hodgkin's disease, anaplastic large-cell lymphoma and Burkitt's lymphoma.

12. A compound or pharmaceutical composition for use according to claim 10 or 11, wherein said compound is administered together, prior to, or subsequently to radiotherapy, immunotherapy or chemotherapy.

13. A compound of formula (II), according to claim 4, for use in the prevention and/or treatment of a bacterial infection.

14. A pharmaceutical composition comprising at least a compound of formula (II), according to any one of claims 4 to 6, and at least one pharmaceutically acceptable excipient or carrier, for use in the prevention and/or treatment of a bacterial infection.

15. A compound for use according to claim 13 or a pharmaceutical composition for use according to claim 14, wherein the bacterial infection is caused by *Staphylococcus aureus.*

## Patentansprüche

1. Verbindung der Formel (I) oder ein pharmazeutisch annehmbares Salz oder Solvat hiervon, 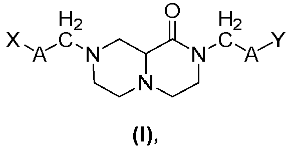 wobei
A jeweils unabhängig voneinander ausgewählt ist aus C1-C6-Alkyl oder nicht vorhanden ist;
X und Y jeweils unabhängig voneinander ausgewählt sind aus C1-C6-Halogenalkyl, einem aromatischen carbocyclischen Sechsring oder einem aromatischen heterocyclischen Fünf- oder Sechsring, der ein oder mehrere Heteroatome ausgewählt aus O, N und S umfasst, wobei der aromatische carbocyclische Ring oder der aromatische heterocyclische Ring unsubstituiert oder an einem oder mehreren Kohlenstoffatomen mit einem R7-Substituenten substituiert ist;
R7 jeweils unabhängig voneinander ausgewählt ist aus H, Halogen, C1-C6-Halogenalkyl, - O-C1-C6-Alkyl, -O-C1-C3-Halogenalkyl, Cyano, C1-C6-Alkyl und -C(O)OR8; und
R8 jeweils unabhängig voneinander ausgewählt ist aus C1-C6-Alkyl, C3-C6-Cycloalkyl und C1-C6-Aminoalkyl.

2. Verbindung der Formel (I) oder ein pharmazeutisch annehmbares Salz oder Solvat hiervon nach Anspruch 1, wobei
A jeweils unabhängig voneinander ausgewählt ist aus C1-C6-Alkyl oder nicht vorhanden ist;
X und Y jeweils unabhängig voneinander ausgewählt sind aus C1-C6-Halogenalkyl, einem aromatischen carbocyclischen Sechsring oder einem aromatischen heterocyclischen Fünf- oder Sechsring, der ein oder mehrere Heteroatome ausgewählt aus O, N und S umfasst, wobei der aromatische carbocyclische Ring oder der aromatische heterocyclische Ring unsubstituiert oder an einem oder mehreren Kohlenstoffatomen mit einem R7-Substituenten substituiert ist;
wobei R7 jeweils unabhängig voneinander ausgewählt ist aus H, Halogen, C1-C6-Halogenalkyl, -O-C1-C6-Alkyl, -O-C1-C2-Halogenalkyl, Cyano und C1-C6-Alkyl.

3. Verbindung der Formel (I) oder ein pharmazeutisch annehmbares Salz oder Solvat hiervon nach Anspruch 1 oder 2, wobei
A jeweils unabhängig voneinander ausgewählt ist aus C1-C3-Alkyl oder nicht vorhanden ist;
X und Y jeweils unabhängig voneinander ausgewählt sind aus C1-C3-Halogenalkyl, einem aromatischen carbocyclischen Sechsring oder einem aromatischen heterocyclischen Fünfring, der ein oder mehrere Heteroatome ausgewählt aus O, N und S umfasst, wobei der aromatische carbocyclische Ring oder der aromatische heterocyclische Ring unsubstituiert oder an einem oder mehreren Kohlenstoffatomen mit einem R7-Substituenten substituiert ist;
wobei R7 jeweils unabhängig voneinander ausgewählt ist aus H, F, Cl, Br, C1-C3-Halogenalkyl, -O-C1-C6-Alkyl, -O-CF3, Cyano und C1-C6-Alkyl.

4. Verbindung nach Anspruch 1 oder 2 mit der Formel (II) oder ein pharmazeutisch annehmbares Salz oder Solvat hiervon, 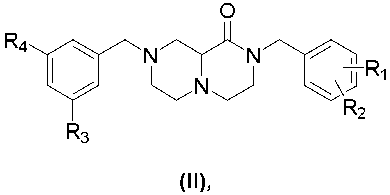 wobei
R1 und R2 jeweils unabhängig voneinander ausgewählt sind aus H, Halogen und CF3;
R3 und R4 jeweils unabhängig voneinander ausgewählt sind aus Halogen und CF3.

5. Verbindung oder pharmazeutisch annehmbares Salz oder Solvat hiervon nach einem der Ansprüche 1 bis 4, wobei die Verbindung ausgewählt ist aus:

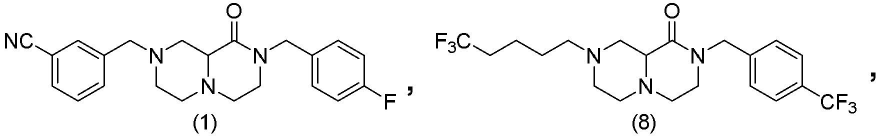

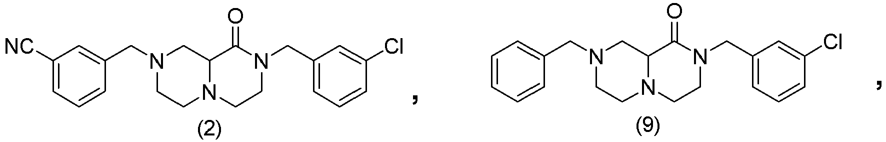

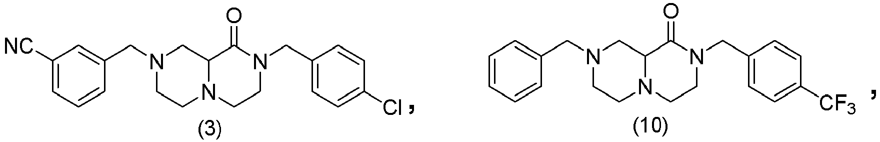

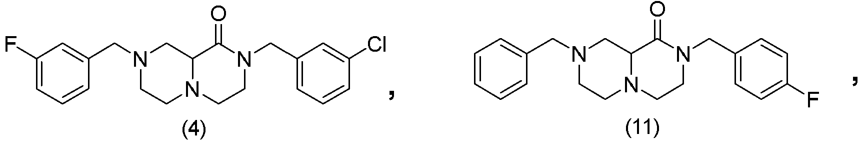

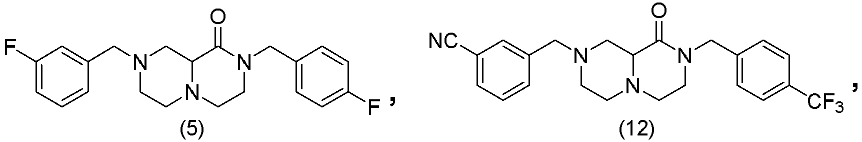

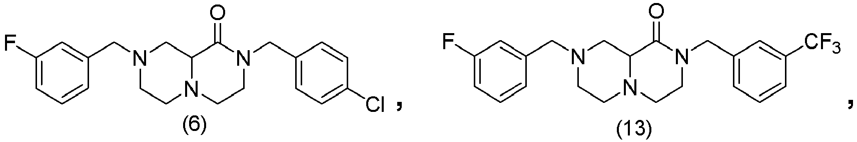

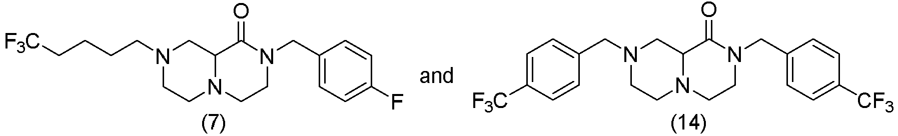

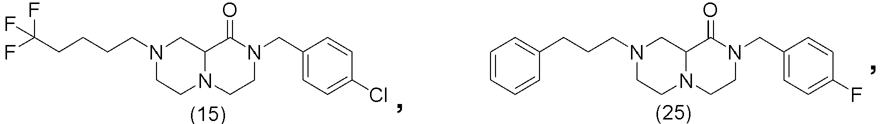

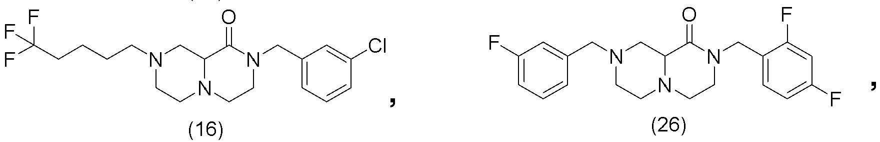

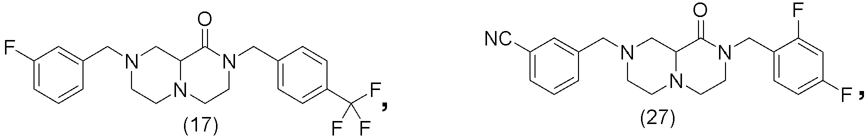

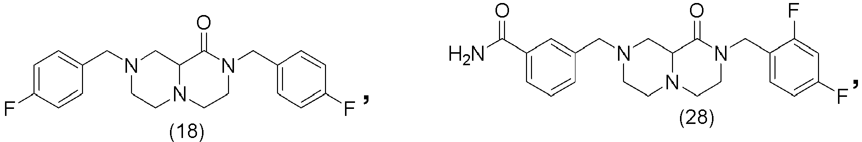

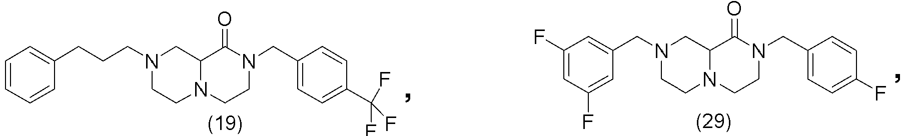

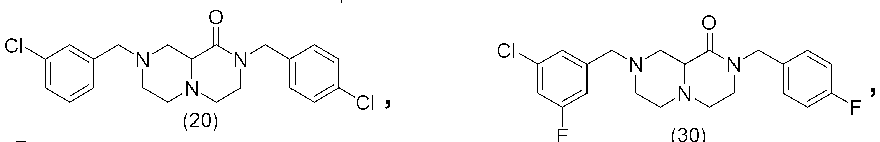

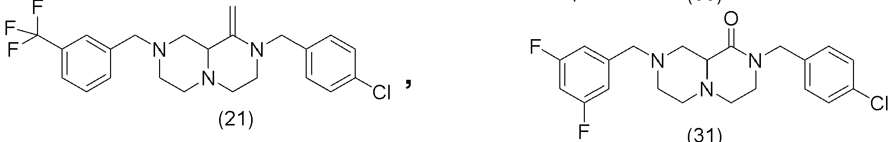

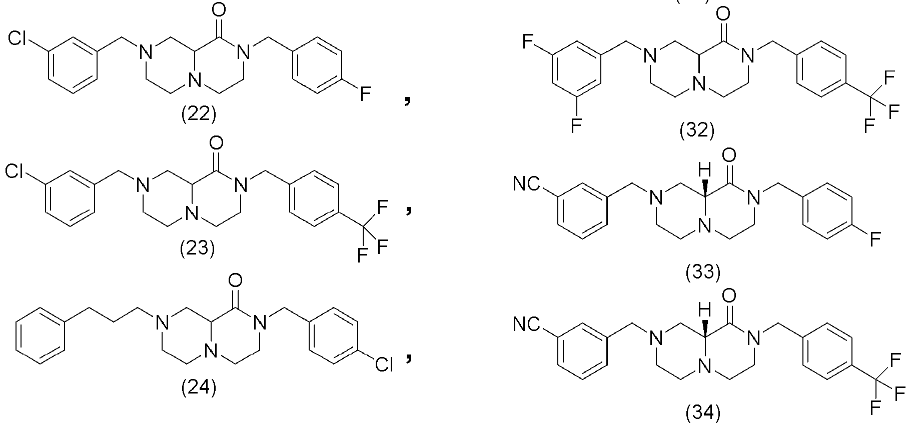

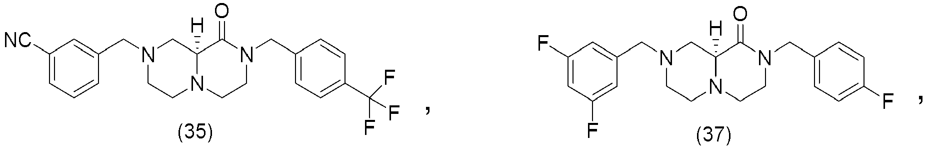

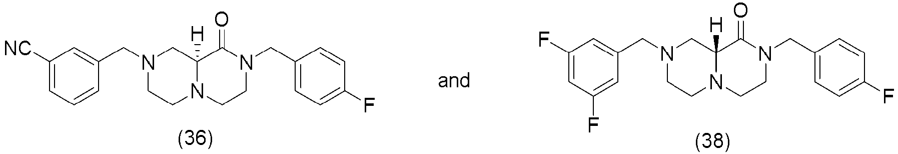

6. Verbindung der Formel (I) oder der Formel (II) oder ein pharmazeutisch annehmbares Salz oder Solvat hiervon nach einem der Ansprüche 1 bis 5, wobei die Verbindung, das Salz oder das Solvat Stereoisomere, Polymorphe oder Isotope umfasst oder in Form von Komplexen mit Antikörpern vorliegt.

7. Pharmazeutische Zusammensetzung, umfassend mindestens eine Verbindung der Formel (I) oder der Formel (II) nach einem der Ansprüche 1 bis 6 und mindestens einen pharmazeutisch annehmbaren Hilfsstoff oder Träger.

8. Pharmazeutische Zusammensetzung nach Anspruch 7, ferner umfassend mindestens eine zusätzliche aktive Verbindung oder ein zusätzliches aktives Mittel, ausgewählt aus einem Chemotherapeutikum, einer Zelltherapie, einer Immuntherapie oder einem Antibiotikum.

9. Verbindung der Formel (I) oder der Formel (II) nach einem der Ansprüche 1 bis 6 oder pharmazeutische Zusammensetzung nach Anspruch 7 oder 8 zur Verwendung als Arzneimittel.

10. Verbindung der Formel (I) oder der Formel (II) nach einem der Ansprüche 1 bis 6 oder pharmazeutische Zusammensetzung nach einem der Ansprüche 7 oder 8 zur Verwendung bei der Prävention und/oder Behandlung von Krebs.

11. Verbindung oder pharmazeutische Zusammensetzung zur Verwendung nach Anspruch 10, wobei der Krebs ausgewählt ist aus Sarkom, Brustkrebs, Prostatakrebs, Kopf- und Halskrebs, Hirntumor, kolorektalem Krebs, Lungenkrebs, Bauchspeicheldrüsenkrebs, Gebärmutterhalskrebs, Eierstockkrebs, Magenkrebs, Nierenzellkarzinom, Melanom, Endometriumkrebs, hepatozellulärem Karzinom, chronischer myeloischer Leukämie, akuter myeloischer Leukämie, kutanem T-Zell-Lymphom, Morbus Hodgkin, anaplastischem großzelligem Lymphom und Burkitt-Lymphom.

12. Verbindung oder pharmazeutische Zusammensetzung zur Verwendung nach Anspruch 10 oder 11, wobei die Verbindung gleichzeitig mit, vor oder nach einer Strahlentherapie, Immuntherapie oder Chemotherapie verabreicht wird.

13. Verbindung der Formel (II) nach Anspruch 4 zur Verwendung bei der Prävention und/oder Behandlung einer bakteriellen Infektion.

14. Pharmazeutische Zusammensetzung, umfassend mindestens eine Verbindung der Formel (II) nach einem der Ansprüche 4 bis 6 und mindestens einen pharmazeutisch annehmbaren Hilfsstoff oder Träger, zur Verwendung bei der Prävention und/oder Behandlung einer bakteriellen Infektion.

15. Verbindung zur Verwendung nach Anspruch 13 oder pharmazeutische Zusammensetzung zur Verwendung nach Anspruch 14, wobei die bakterielle Infektion durch *Staphylococcus aureus* verursacht wird.

## Revendications

1. Composé de formule (I), ou un sel pharmaceutiquement acceptable ou un solvate de celui-ci, 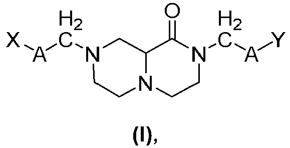 dans lequel
A est, pour chaque occurrence, choisi indépendamment parmi un alkyle en C₁-C₆ ou est absent ;
X et Y sont, pour chaque occurrence, choisis indépendamment parmi un halogénoalkyle en C1-C6, un cycle carbocyclique aromatique à six chaînons ou un cycle hétérocyclique aromatique à cinq ou six chaînons comportant un ou plusieurs hétéroatomes choisis parmi O, N et S, ledit cycle carbocyclique aromatique ou ledit cycle hétérocyclique aromatique étant non substitué ou substitué sur un ou plusieurs atomes de carbone par un substituant R7 ;
R7 est, pour chaque occurrence, choisi indépendamment parmi **H,** un halogène, un halogénoalkyle en C1-C6, -O-C1-C6 alkyle, -O-C1-C3 haloalkyle, cyano, un alkyle en C1-C6 et -C(O)OR8 ; et
R8 est, pour chaque occurrence, choisi indépendamment parmi un alkyle en C1-C6, un cycloalkyle en C3-C6 et un aminoalkyle en C1-C6.

2. Composé de formule (I), ou un sel pharmaceutiquement acceptable ou un solvate de celui-ci, selon la revendication 1, dans lequel
A est, pour chaque occurrence, choisi indépendamment parmi un alkyle en C1-C6 ou est absent;
X et Y sont, pour chaque occurrence, choisis indépendamment parmi un halogénoalkyle en C1-C6, un cycle carbocyclique aromatique à six chaînons ou un cycle hétérocyclique aromatique à cinq ou six chaînons comportant un ou plusieurs hétéroatomes choisis parmi O, N et S, ledit cycle carbocyclique aromatique ou ledit cycle hétérocyclique aromatique étant non substitué ou substitué sur un ou plusieurs atomes de carbone par un substituant R7 ;
dans lequel R7 est, pour chaque occurrence, choisi indépendamment parmi H, un halogène, un halogénoalkyle en C1-C6, -O-C1-C6 alkyle, -O-C1-C2 haloalkyle, cyano et un alkyle en C1-C6.

3. Composé de formule (I), ou un sel pharmaceutiquement acceptable ou un solvate de celui-ci, selon la revendication 1 ou 2, dans lequel
A est, pour chaque occurrence, choisi indépendamment parmi un alkyle en C1-C3 ou est absent;
X et Y sont, pour chaque occurrence, choisis indépendamment parmi un halogénoalkyle en C1-C3, un cycle carbocyclique aromatique à six chaînons ou un cycle hétérocyclique aromatique à cinq chaînons comportant un ou plusieurs hétéroatomes choisis parmi O, N et S, ledit cycle carbocyclique aromatique ou ledit cycle hétérocyclique aromatique étant non substitué ou substitué sur un ou plusieurs atomes de carbone par un substituant R7 ;
dans lequel R7 est, pour chaque occurrence, choisi indépendamment parmi H, F, Cl, Br, un halogénoalkyle en C1-C3, -O-C1-C6 alkyle, -O-CF3, cyano et un alkyle en C1-C6.

4. Composé selon la revendication 1 ou 2, répondant à la formule (II), ou un sel pharmaceutiquement acceptable ou un solvate de celui-ci, 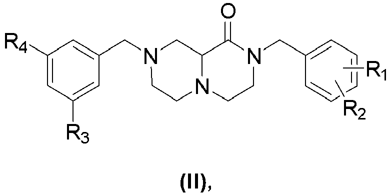 dans lequel
R1 et R2 sont, pour chaque occurrence, choisis indépendamment parmi H, un halogène et CF3 ;
R3 et R4 sont, pour chaque occurrence, choisis indépendamment parmi un halogène et CF3.

5. Composé, ou un sel pharmaceutiquement acceptable ou un solvate de celui-ci, selon l'une quelconque des revendications 1 à 4, dans lequel le composé est choisi parmi :

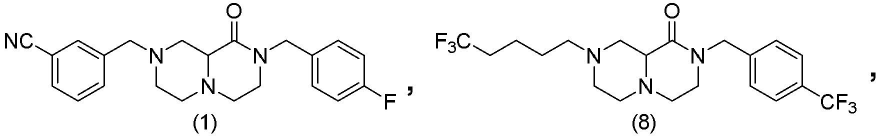

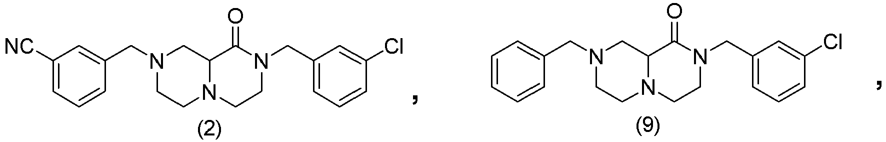

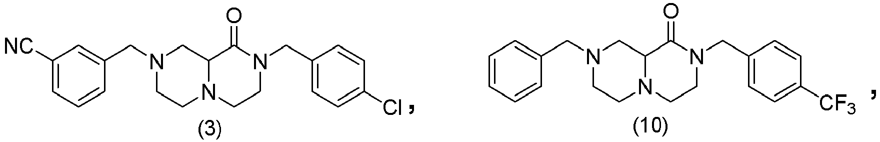

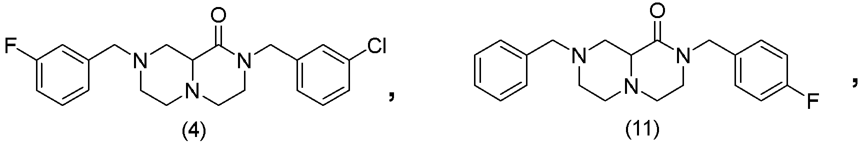

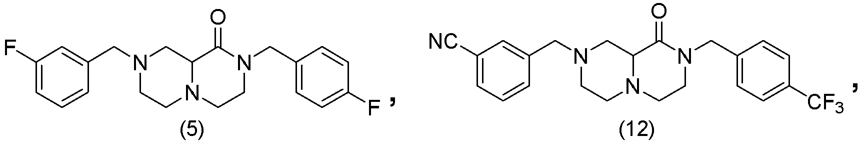

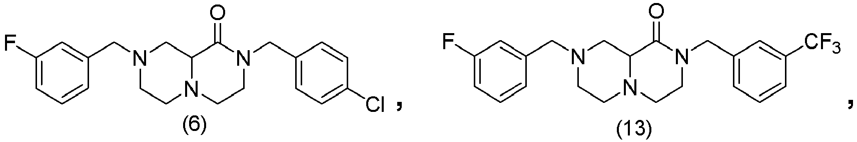

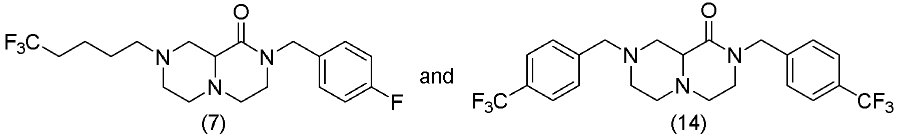

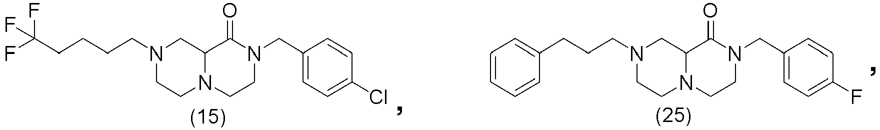

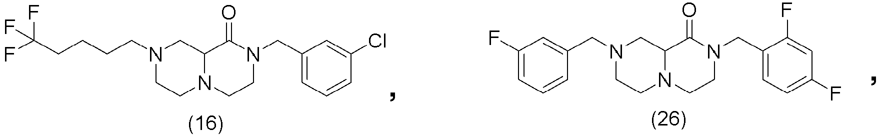

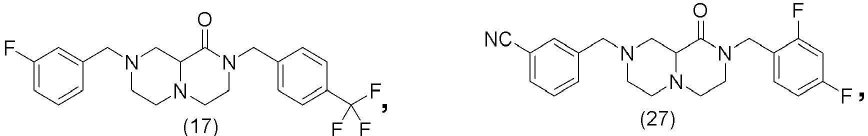

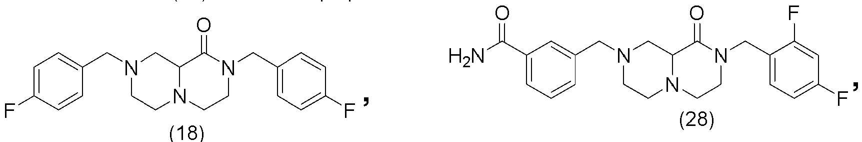

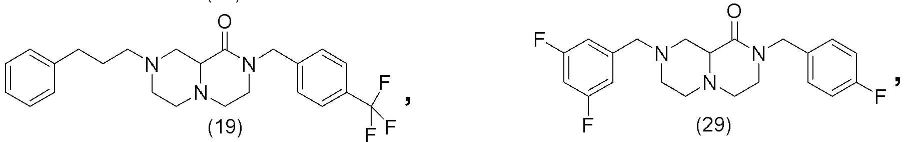

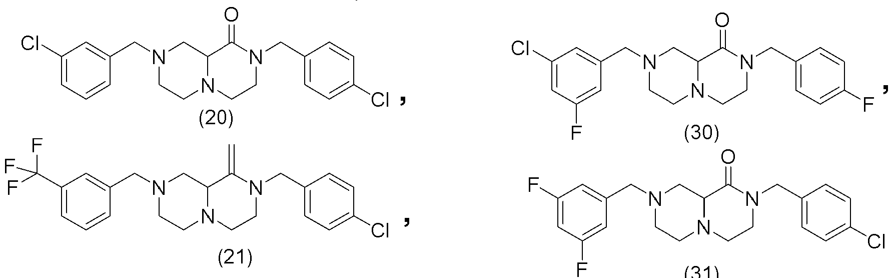

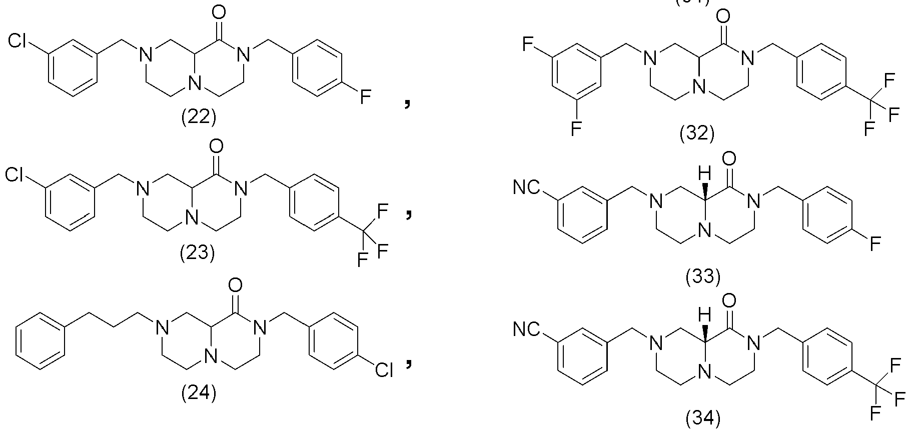

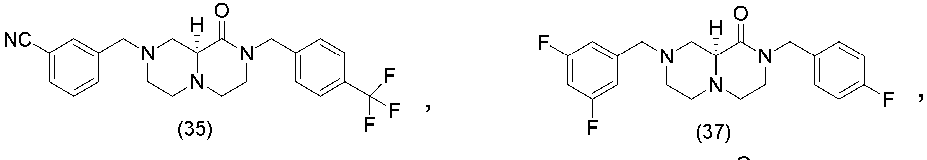

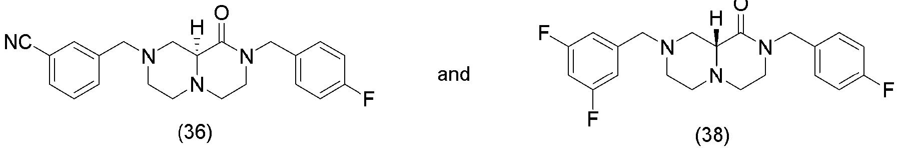

6. Composé de formule (I) ou de formule (II), ou un sel pharmaceutiquement acceptable ou un solvate de celui-ci, selon l'une quelconque des revendications 1 à 5, dans lequel le composé, le sel ou le solvate comprend des stéréoisomères, des polymorphes, des isotopes, ou est sous forme de complexes avec des anticorps.

7. Composition pharmaceutique comprenant au moins un composé de formule (I) ou de formule (II) selon l'une quelconque des revendications 1 à 6, et au moins un excipient ou véhicule pharmaceutiquement acceptable.

8. Composition pharmaceutique selon la revendication 7, comprenant en outre au moins un composé actif supplémentaire ou un agent actif supplémentaire choisi parmi un agent chimiothérapeutique, une thérapie cellulaire, une immunothérapie ou un antibiotique.

9. Composé de formule (I) ou de formule (II) selon l'une quelconque des revendications 1 à 6, ou composition pharmaceutique selon la revendication 7 ou 8, pour une utilisation en tant que médicament.

10. Composé de formule (I) ou de formule (II) selon l'une quelconque des revendications 1 à 6, ou composition pharmaceutique selon l'une quelconque des revendications 7 ou 8, pour une utilisation dans la prévention et/ou le traitement du cancer.

11. Composé ou composition pharmaceutique pour une utilisation selon la revendication 10, dans lequel le cancer est choisi parmi un sarcome, un cancer du sein, un cancer de la prostate, un cancer de la tête et du cou, une tumeur cérébrale, un cancer colorectal, un cancer du poumon, un cancer du pancréas, un cancer du col de l'utérus, un cancer de l'ovaire, un cancer gastrique, un carcinome à cellules rénales, un mélanome, un cancer de l'endomètre, un carcinome hépatocellulaire, une leucémie myéloïde chronique, une leucémie myéloïde aiguë, un lymphome cutané à cellules T, la maladie de Hodgkin, un lymphome anaplasique à grandes cellules et un lymphome de Burkitt.

12. Composé ou composition pharmaceutique pour une utilisation selon la revendication 10 ou 11, dans lequel ledit composé est administré conjointement avec, avant ou après une radiothérapie, une immunothérapie ou une chimiothérapie.

13. Composé de formule (II) selon la revendication 4, pour une utilisation dans la prévention et/ou le traitement d'une infection bactérienne.

14. Composition pharmaceutique comprenant au moins un composé de formule (II) selon l'une quelconque des revendications 4 à 6, et au moins un excipient ou véhicule pharmaceutiquement acceptable, pour une utilisation dans la prévention et/ou le traitement d'une infection bactérienne.

15. Composé pour une utilisation selon la revendication 13 ou composition pharmaceutique pour une utilisation selon la revendication 14, dans lequel l'infection bactérienne est causée par *Staphylococcus aureus.*
